# EUROPEAN PATENT APPLICATION

(11) **EP 3 168 310 A1**
(43) Date of publication of application: **17.05.2017**
(21) Application number: 15194419.6
(22) Date of filing: 13.11.2015
(51) Int. Cl.: C12Q 1/68, A61K 31/7068

(54) **METHYLATION MARKERS FOR COLORECTAL CANCER**

(71) Applicant: Academisch Medisch Centrum, 1105 AZ Amsterdam (NL)
(72) Inventor: The designation of the inventor has not yet been filed
(74) Representative: de Pauw, Elmar Sebastian David

(57) **Abstract**

The present invention provides a method of diagnosing, prognosticating, predicting or detecting pre-cancer, colorectal cancer (CRC) tumor progression, or metastasis in a human subject comprising the steps of:
(a) determining in a biological sample from said human subject the methylation status of at least one CpG in the miR-200ba429 promoter region and/or the miR-200c141 promoter region, and
(b) determining the methylation status of one or more of said at least one CpG,
(c) determining the risk of developing CRC, metastasis and/or tumor relapse based on said methylation status, wherein hypermethylation of said at least one CpG indicates an increased risk of developing CRC, metastasis and/or tumor relapse.

SEQUENCE LISTING

<110> Academisch Medisch Centrum

<120> METHYLATION MARKERS FOR COLORECTAL CANCER

<130> 2015-039

<160> 12

<170> BiSSAP 1.2

<210> 1
<211> 26
<212> DNA
<213> Artificial Sequence

<220>
<221> source
<222> 1..26
<223> /organism="Artificial Sequence"
      /note="miR-200ba429 FW primer"
      /mol_type="unassigned DNA"

<400> 1
   ggtttgaatt gattttttgt gttagg                                          26

<210> 2
<211> 22
<212> DNA
<213> Artificial Sequence

<220>
<221> source
<222> 1..22
<223> /organism="Artificial Sequence"
      /note="miR-200ba429 RV primer"
      /mol_type="unassigned DNA"

<400> 2
cctcaaccaa aatcaaacct ca                                             22

<210> 3
<211> 19
<212> DNA
<213> Artificial Sequence

<220>
<221> source
<222> 1..19
<223> /organism="Artificial Sequence"
      /note="miR-200ba429 Seq primer"
      /mol_type="unassigned DNA"

<400> 3
   attttttgtg ttagggttt                                                  19

<210> 4
<211> 19
<212> DNA

<213> Artificial Sequence

<220>
<221> source
<222> 1..19
<223> /organism="Artificial Sequence"
      /note="miR-200c141 FW primer"
      /mol_type="unassigned DNA"

<400> 4
attgtagagg ggggatgag                                                    19


<210> 5
<211> 23
<212> DNA
<213> Artificial Sequence

<220>
<221> source
<222> 1..23
<223> /organism="Artificial Sequence"
      /note="miR-200c141 RV primer"
      /mol_type="unassigned DNA"

<400> 5
ccaaattaca atccaaacaa acc                                               23


<210> 6
<211> 15
<212> DNA
<213> Artificial Sequence

<220>
<221> source
<222> 1..15
<223> /organism="Artificial Sequence"
      /note="miR-200c141 Seq primer"
      /mol_type="unassigned DNA"

<400> 6
gatgagggtg ggtaa                                                        15


<210> 7
<211> 20
<212> DNA
<213> Artificial Sequence

<220>
<221> source
<222> 1..20
<223> /organism="Artificial Sequence"
      /note="GAPDH FW primer"
      /mol_type="unassigned DNA"

<400> 7
aatcccatca ccatcttcca                                                   20


<210> 8
<211> 21

<212> DNA
<213> Artificial Sequence

<220>
<221> source
<222> 1..21
<223> /organism="Artificial Sequence"
      /note="ZEB1 FW primer"
      /mol_type="unassigned DNA"

<400> 8
gcacaagaag agccacaagt a                                                21


<210> 9
<211> 20
<212> DNA
<213> Artificial Sequence

<220>
<221> source
<222> 1..20
<223> /organism="Artificial Sequence"
      /note="E-Cadherin FW primer"
      /mol_type="unassigned DNA"

<400> 9
tggaggaatt cttgctttgc                                                  20


<210> 10
<211> 20
<212> DNA
<213> Artificial Sequence

<220>
<221> source
<222> 1..20
<223> /organism="Artificial Sequence"
      /note="GAPDH RV primer"
      /mol_type="unassigned DNA"

<400> 10
tggactccac gacgtactca                                                  20


<210> 11
<211> 21
<212> DNA
<213> Artificial Sequence

<220>
<221> source
<222> 1..21
<223> /organism="Artificial Sequence"
      /note="ZEB1 RV primer"
      /mol_type="unassigned DNA"

<400> 11
gcaagacaag ttcaagggtt c                                                21


<210> 12

<211> 19
<212> DNA
<213> Artificial Sequence

<220>
<221> source
<222> 1..19
<223> /organism="Artificial Sequence"
      /note="E-Cadherin RV primer"
      /mol_type="unassigned DNA"

<400> 12
cgctctcctc cgaagaaac                                                    19

## Description

### TECHNICAL FIELD OF THE INVENTION

The present invention relates in general to the use of the methylation status and or the expression level of particular genes and their potential to predict the recurrence of colorectal cancer or predict drug resistance/susceptibility. More specifically, the invention relates to the methylation status of a CpG in the promotor region of a gene the miR-200 family members as a biomarker for prognosis and treatment of colorectal cancer.

### BACKGROUND OF THE INVENTION

Colon cancer is a heterogeneous disease, which until recently has only been reflected by histopathological classification based on tumor stage and differentiation grade, and by single molecular markers such as microsatellite instability (MSI) and mutation status of major tumor suppressor genes and oncogenes. However, the diversity of the disease has made it difficult to accurately classify and treat colon cancer patients. Only lately, colon cancers have been categorized using unsupervised classification of gene expression profiling which resulted in the identification of distinct colon cancer subtypes (CCSs) (1-6). Different CCSs associate with specific molecular, biological, and clinical features and thus present distinct entities. Linking the subtypes to disease outcome revealed a previously unidentified poor prognosis CCS, highlighting the clinical relevance of this approach (2). One subtype which is associated with a poor prognosis is characterized by an epithelial-to-mesenchymal transition. Patients having this subtype receive no benefit from adjuvant chemotherapy treatment with for example fluorouracil (5-FU).
The current technology that we and others have used to identify the subgroups is full gene expression profiling De Sousa et al., Nature Medicine 2013. Even though this allows for the identification of patients, it only works when a bigger group is analyzed in one go and patients can be compared to each other and more importantly, it is a very laborious and costly method. Another drawback of the known gene profiles used for classification of the different subtypes is that they have little or no prognostic value, as they are not reliable.
There is therefore a need for subtype-specific markers which do not have one or more of these drawbacks.
Cancer cells undergoing EMT lose epithelial characteristics, such as polarity and cell-cell-adhesion, and gain mesenchymal features that allow them to leave the primary tumor, enter the blood stream, and disseminate to distant organs (*15*). Thus, the EMT program is commonly thought to occur late in tumor progression and its presence marks aggressive and invasive tumor cells and subtypes. A direct link between miRNAs and EMT was firmly established and specifically the miR-200 family has been implicated in repressing the EMT zinc finger homeodomain enhancer-binding (ZEB) transcription factors ZEB1 and ZEB2 (*16*, *17*), thereby inhibiting the EMT program. The expression of the miR-200 family members and consequently the activity of the EMT process are determined by promoter methylation of the miR-200 loci (*18-21*).
Here we report a regulatory network consisting of methylated miR-200 family promoter regions and low expression of miR-200 family members responsible for tuning the gene expression of poor prognosis colon cancers, which is already installed in a defined subset of precursor lesions. We show that in colon cancer the miR-200 family cannot only be used to define distinct cellular states, such as associated with the EMT program, but regulates subtype-specific gene expression thus being a defining factor for the subset of poor prognosis colon cancers. Furthermore, we show that the epigenetic methylation mark that inhibits the network can be used to identify patients belonging to the mesenchymal subtype at early stages in tumor development and is a prognostic determinant of colon cancer.

### SUMMARY OF THE INVENTION

The present invention is based, in part, on the use of methylation profiling to show that methylation biomarkers are capable of classifying epithelial and mesenchymal phenotypes in colorectal cancer, demonstrating that genome -wide differences in DNA methylation patterns are associated with distinct biologic and clinically relevant subsets of that cancer. The results by the inventors clearly show this: Figure 1 shows that colon cancer samples can be classified into three distinct subtypes based on gene expression (CCS1 to CCS3). Figure 4 shows that high promoter methylation in CCS3 tumors and sessile serrated adenomas (SSAs) is correlated with low expression of the miR-200 family members. Figure 5 shows that CCS3 cell lines often display high miR-200 promoter methylation levels, which are instrumental for miR-200 expression. Figure 6 shows that miR-200 loci methylation is predictive of CCS3 affiliation and prognosis.

The use of methylation patterns to classify phenotypic subsets of cancers using the methods described herein is advantageous as it requires less quantity of test tissue as compared to more traditional methods of DNA- and RNA-based analyses. This feature is particularly useful when analyzing clinical samples where tissue is limited.

The miR-200 family of microRNAs has been previously shown to regulate EMT genes, which is a process that is thought of as intimately linked to metastases formation. As far as the inventors are aware thereof, it is unknown that methylation of these miRNAs is predictive of CRC progression and certainly not at an early stage of CRC development as the inventors have found. Most of these studies of the role of miR-200 family in cancer progression have focused on breast cancer and there is evidence that the miR-200 family can regulate the transition from the epithelial to mesenchymal state. For colon cancers, the involvement of miR-200 family in cancer progression remains elusive. US2012264131 (A1) discloses a method for diagnosing colorectal cancer by determining the overall expression pattern or level of one or more microRNAs from the miR-200 family, but does not disclose the methylation of these miRNAs. The inventors show that this also holds true in colon cancer and more importantly, that the miR-200 family cannot only be used to define distinct cellular states, but regulates subtype-specific gene expression and thus is able to identify the subtype affiliation of colon cancer samples.

Critical to appreciate the importance of the current work is the notion that in recent years it has been established that colon cancer is in fact a collection of diseases that are extensively different. A series of studies by us (De Sousa et al., Cell Stem Cell, 2011; De Sousa et al., Nature Medicine 2013), and others (e.g. TCGA, Nature 2010; Schlicker et al., BMC medical genomics 2012, Sadanandam et al., Nature Medicine 2013; Budinska et al., The journal of pathology 2013, Marisa et al., PLoS Medicine 2013, Roepman et al., International journal of cancer 2014) have used unbiased gene expression based strategies to identify these subtypes. In all these reports a distinct, mesenchymal-like subtype was recognized with dismal prognosis, which makes it of utmost importance to characterize this subtype. This notion is further enforced by the realization that currently used therapies are much less effective in this subtype, and it will be important to develop and/or test novel agents to improve management of these patients. What has remained elusive however is what explains the distinction in subtypes as all studies have reported that individual mutations, or in fact combinations of mutations, do not define the subtypes. Furthermore, tools to easily identify the mesenchymal colon cancer subtype are lacking and the current gold-standard detection method using comprehensive gene expression data to classify patients is both impractical and importantly, it does not allow for classification of individual patients. The biomarkers of the present invention overcome such disadvantages. The inventors validated the presence of colon cancer subtypes, including the poor prognosis mesenchymal subtype, in a new and large colon cancer dataset. They discovered that it is an epigenetic network that prominently involves the miR-200 family that defines this subtype. The miR-200 family of miRs not only regulates EMT/mesenchymal genes, but actually a very large part of the genes that are specific for the colon cancer subtype distinction (∼70% of differential genes). They report that methylation of the miR-200 family is the most upstream regulatory element, and this feature is already present in precursor lesions that relate to the mesenchymal subtype (sessile serrated adenomas). This in turn demonstrates that poor prognosis cancers have a different developmental trajectory and could be already primed for metastatic spread early in their development, even at the premalignant state. The invention therefore provides an easy and validated approach to identify the mesenchymal/poor prognosis subtype in patient material using methylation biomarkers.

In addition, the biomarkers may also be used as tools for evaluating treatment strategies of colorectal cancer patients. Since a poor disease outcome is a feature of mesenchymal phenotype subtype, the methylation biomarker is also of relevance in identifying patients with a dismal prognosis. In the particular case of stage II colon cancer that the inventors focused on, it is actually of relevance to identify patients at higher risk of recurrence following surgery. Because of the overall good disease outcome of this population (80-85% of patients are cured after surgery alone) these patients do generally not receive adjuvant therapy. However, the results of the present invention demonstrate that already at this early stage it is possible to detect a subset of patients displaying a much poorer outcome. This fraction can be identified by high miR-200 promoter methylation levels and could potentially benefit from adjuvant treatment.

Furthermore, the newly discovered methylations markers may be used in methods to identify patients of the mesenchymal subtype to characterize them in-depth. The invention provides a method of diagnosing, prognosticating, predicting or detecting pre-cancer, colorectal cancer (CRC) tumor progression, or metastasis in a human subject comprising the steps of:
(a) determining in a biological sample from said human subject the methylation status of at least one CpG in the miR-200ba429 promoter region and/or the miR-200c141 promoter region, and
(b) determining the methylation status of one or more of said at least one CpG,
(c) determining the risk of developing CRC, metastasis and/or tumor relapse based on said methylation status, wherein hypermethylation of said at least one CpG indicates an increased risk of developing CRC, metastasis and/or tumor relapse.

In another aspect, the invention provides a method of determining whether a colorectal cancer cell has an epithelial or mesenchymal phenotype comprising the steps of:
(a) determining in a biological sample from said human subject the methylation status of at least one CpG in the in the miR-200ba429 promoter region and/or the miR-200c141 promoter region, and
(b) determining the methylation status of one or more of said at least one CpG,
(c) determining the cancer cells as having epithelial or mesenchymal phenotype based on said methylation status, wherein hypermethylation of said at least one CpG indicates a mesenchymal phenotype.
In aspects of the invention, it is preferred that said said methylation status comprises the presence or absence of methylation of DNA at said at least one CpG.
In a preferred embodiment according to the methods of the invention, said biological sample comprises cancer cells from a primary tumor. Preferably, hypermethylation is determined in a CpG of the miR-200ba429 promoter region and in a CpG of the miR-200c141 promoter region. In preferred embodiments according to the methods of the invention said human subject is suffering from colorectal cancer in stage 0, I, II, III or IV. In another preferred embodiment, said human subject is suffering from colorectal cancer in stage I or II. In another preferred embodiment, said human subject is suffering from colorectal cancer in stage II or III.
In another aspect, the invent further provides a method of predicting the response in a human subject to treatment with a demethylating agent comprising:
(a) determining the risk of developing metastasis and/or tumor relapse in human subject according to the method of the invention described above or the phenotype of a cancer cells in a sample from said colorectal cancer patient according to the method described above, and
(b) predicting the clinical response to said treatment, based on the outcome of step (a).
Further provide is a demethylating agent for use in the treatment in the treatment of human subject with hypermethylation of the miR-200ba429 promoter region and/or the miR-200c141 promoter region. Preferably, said demethylating agent is 5-aza-cytidine, 5-aza-2'-deoxycytidine (decitabine), zebularine [1-(β-D-ribofuranosyl(dihydro-pyrimidin-2-1)] or L-methionine.
The invention further provides a kit for assessing methylation in a test sample, comprising in a package:
A reagent that (a) modifies methylated cytosine residues but not non-methylated cytosine residues, or that (b) modifies non-methylated cytosine residues but not methylated cytosine residues; and one or more oligonucleotide primers and/or pair of oligonucleotide primers that specifically hybridizes under amplification conditions to a nucleic acid selected from the group consisting of the miR-200ba429 promoter region and/or the miR-200c141 promoter region.

### BRIEF DESCRIPTION OF THE FIGURES

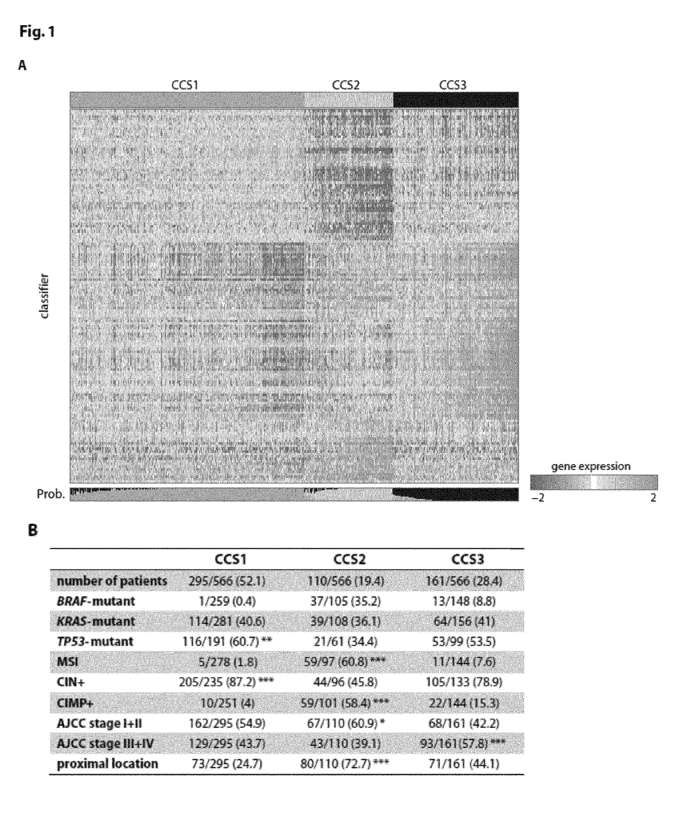

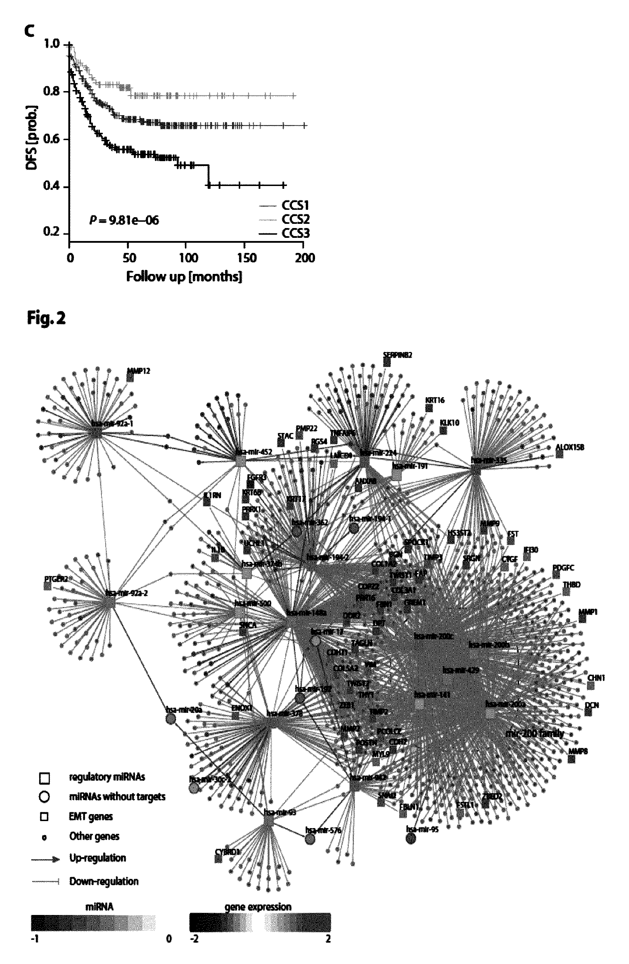

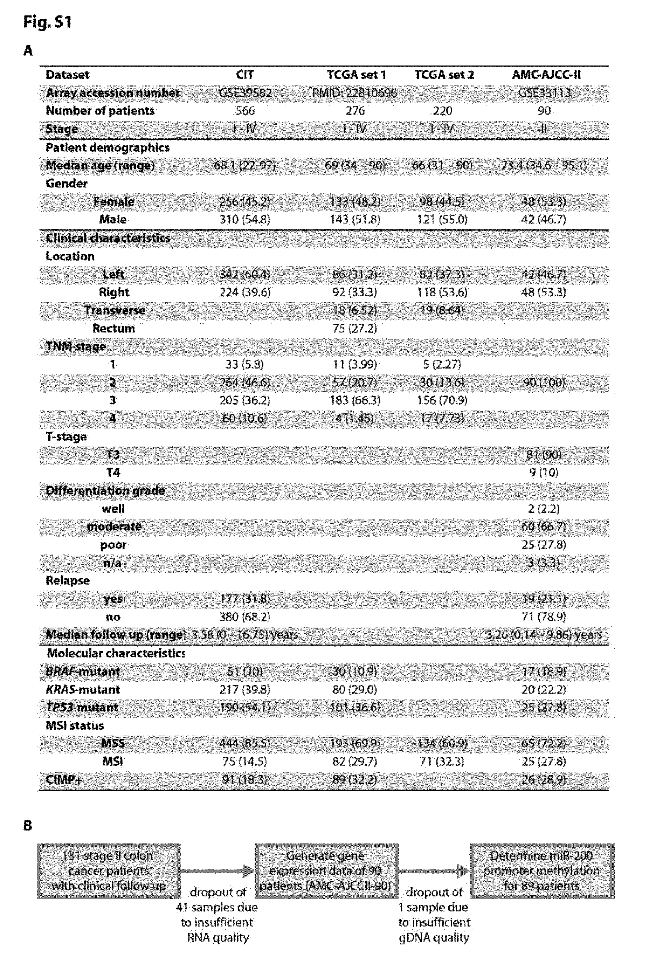

Figure 1 shows that colon cancer samples can be classified into three distinct subtypes based on gene expression. (A) 566 patients of the CIT dataset are classified in three CCSs. Columns indicate patients, whose subtype is indicated in the top bar. The posterior probability of belonging to the subtype is shown in the bar below the heatmap. Rows represent genes of a 146-gene classifier. The heatmap displays the median-centered log2 gene expression levels (high expression: orange, low expression: blue). (B) Association of each subtype with molecular characteristics as well as stage and tumor location. The number of patients displaying the respective characteristic as well as the total number of patients analyzed is shown. The percentage of positivity is shown in brackets. Asterisks indicate the significance of association of one subtype with the respective feature as determined by hypergeometric tests (*P < 0.05, **P < 0.01, ***P < 0.001). (C) KM curve showing DFS of patients of the CIT set according to CCS subtype. P-value is based on log-rank test.

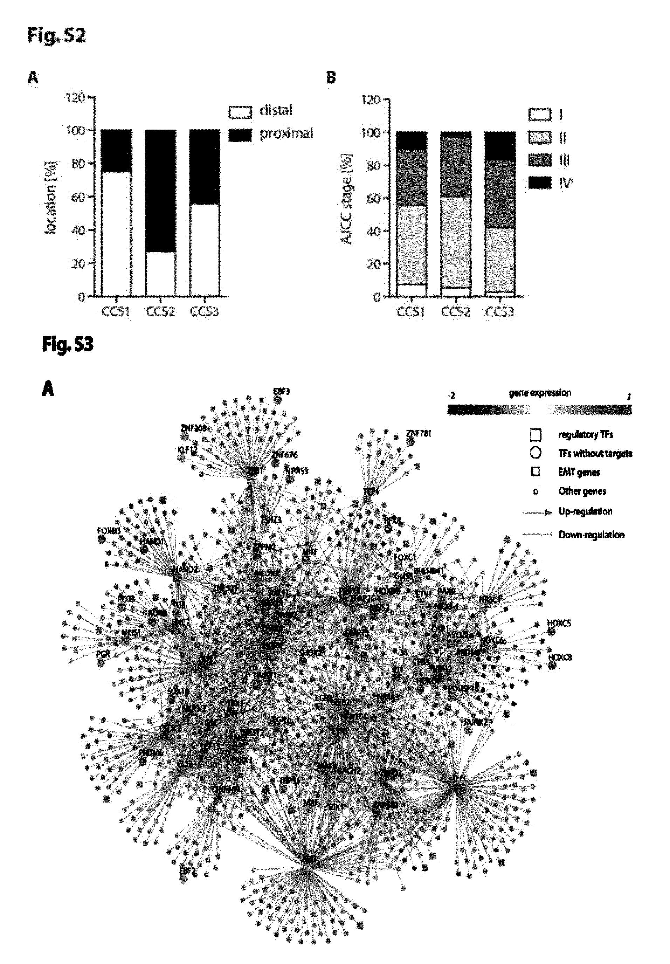

Figure 2 shows that network analysis identifies the miR-200 family as main regulatory network of CCS3- and EMT-related genes. Displayed is the differential miRNA expression between CCS1 and CCS3 tumors of the TCGA dataset (green: lowly expressed in CCS3, white: highly expressed in CCS3). Genes predicted to be regulated by the miRNAs are shown based on their differential expression between CCS1 and CCS3 tumors (blue: lowly expressed in CCS3, orange: highly expressed in CCS3). The connection between miRNAs and genes is depicted in red (induction) or blue (repression) based on the influence of the miRNA on gene expression. EMT-associated genes from the Taube EMT signature are highlighted as rectangles.

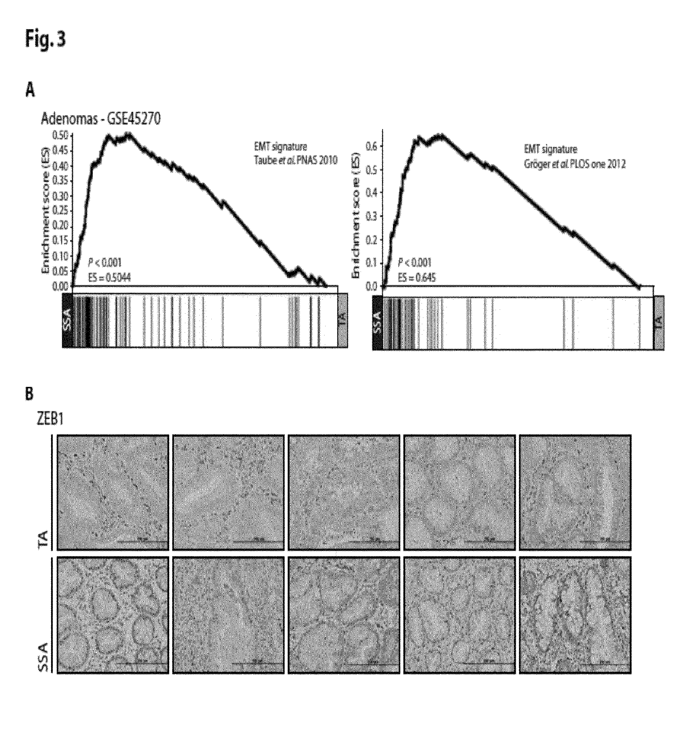

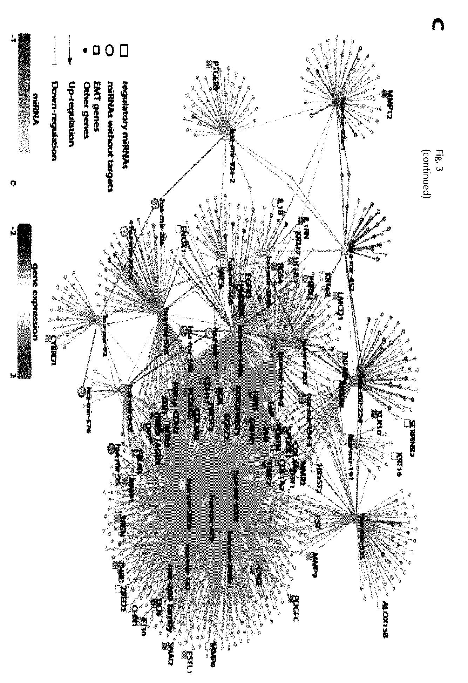

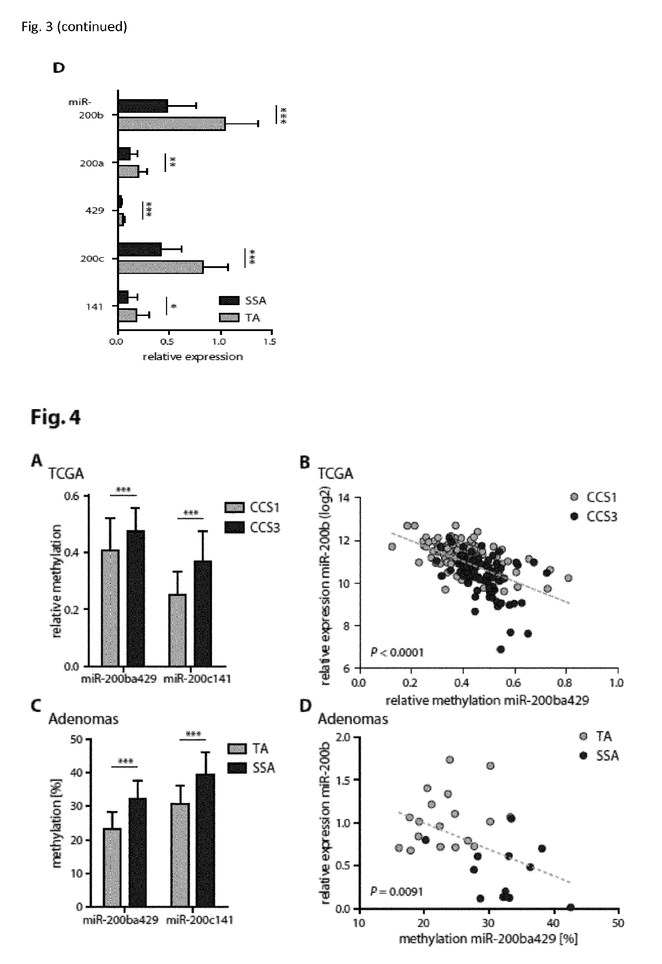

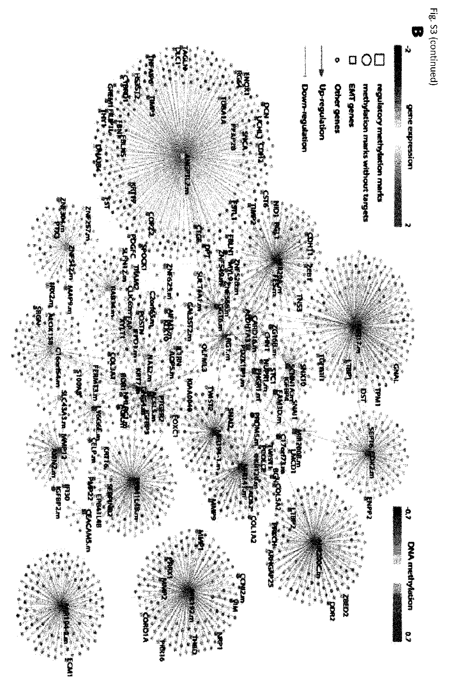

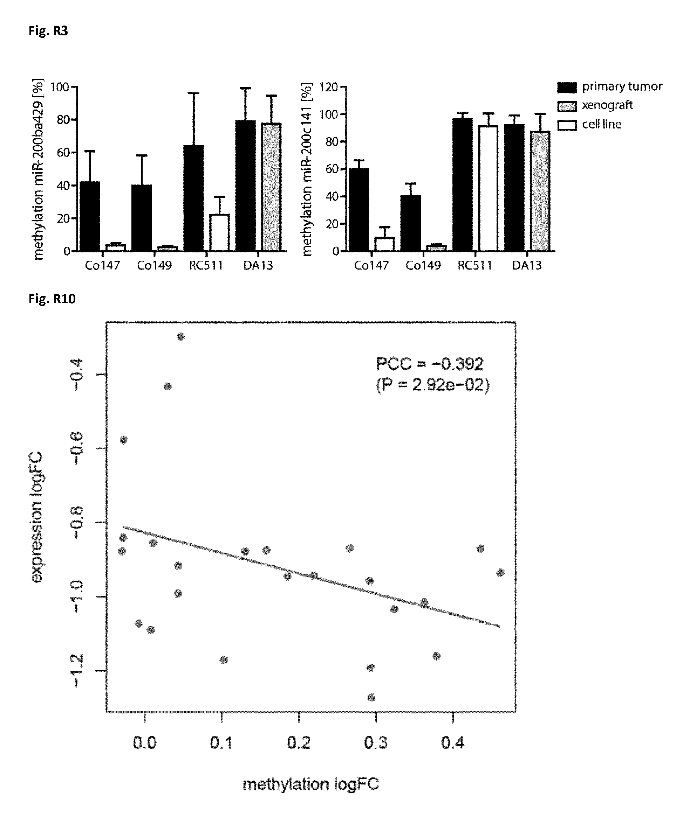

Figure 3 shows that sessile serrated adenomas (SSA) display high expression of EMT-associated genes regulated by miR-200 family members. (A) GSEA using two EMT signatures (Taube et al. 2010, Gröger et al. 2012) applied to a gene expression dataset comprising 6 SSAs and 7 TAs (ES = enrichment score). (B) Immunohistochemical staining of ZEB1 on TA and SSA samples. (C) Differential gene expression data of 6 SSAs and 7 TAs is projected on the network shown in Figure 2. Most genes regulated by the miRNAs lowly expressed in CCS3 tumors are highly expressed in SSA polyps. (D) Detection of miR-200 family members by quantitative real-time PCR in 18 TA and 21 SSA samples (*P < 0.05, **P < 0.01, ***P < 0.001; P-values are based on two-tailed Student's t-tests).

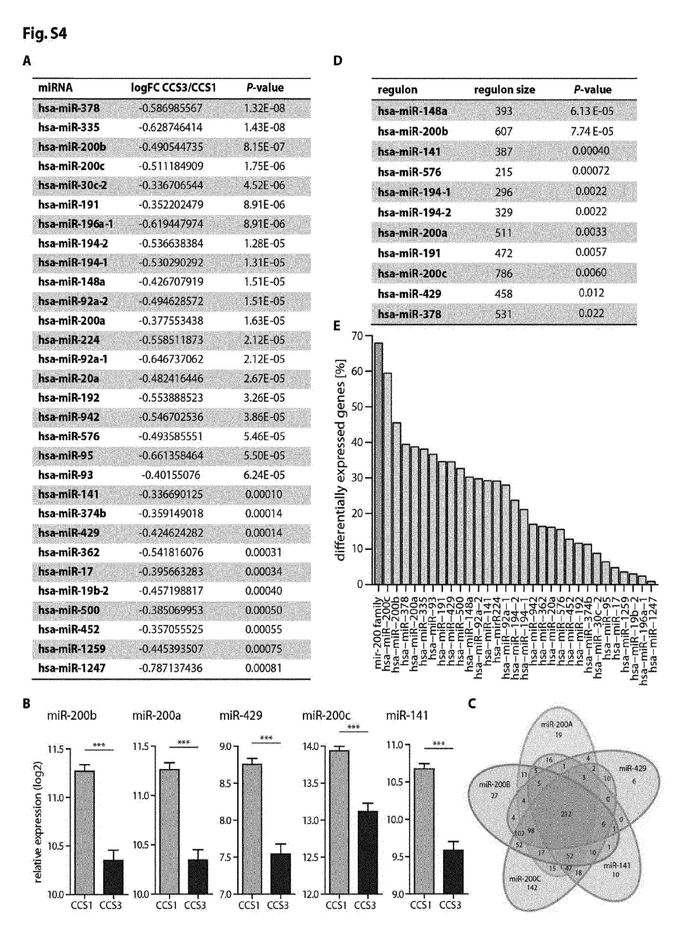

Figure 4 shows that high promoter methylation in CCS3 tumors and SSAs is correlated with low expression of the miR-200 family members. (A) Relative methylation of the miR-200c141 and miR-200ba429 locus in CCS1 and CCS3 samples of the TCGA dataset. Shown is the average methylation of the two/three miRNAs taken together (n=101 for CCS1 and n=80 for CCS3, P < 0.001; P-values are based on two-tailed Student's t-tests). (B) The relative methylation of the miR-200ba429 locus is highly correlated with the expression of miR-200b. (C) Methylation of the miR-200ba429 and miR-200c141 promoter regions in adenoma samples determined by pyrosequencing (n=18 for TA and n=13 for SSA, P < 0.001; P-values are based on two-tailed Student's t-tests). (D) The methylation of the miR-200ba429 locus correlates with the expression of miR-200b.

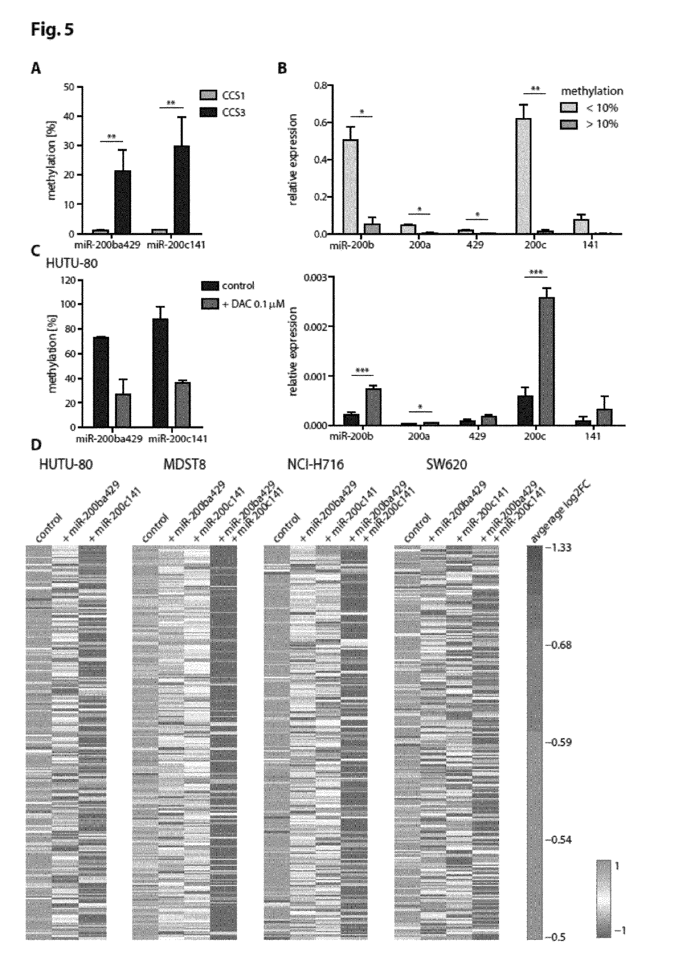

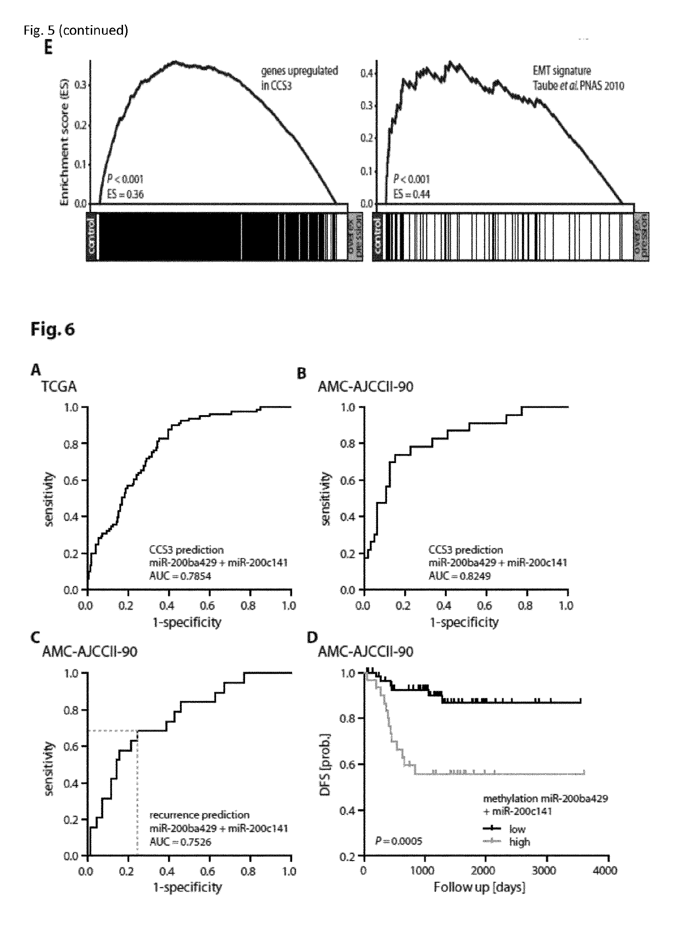

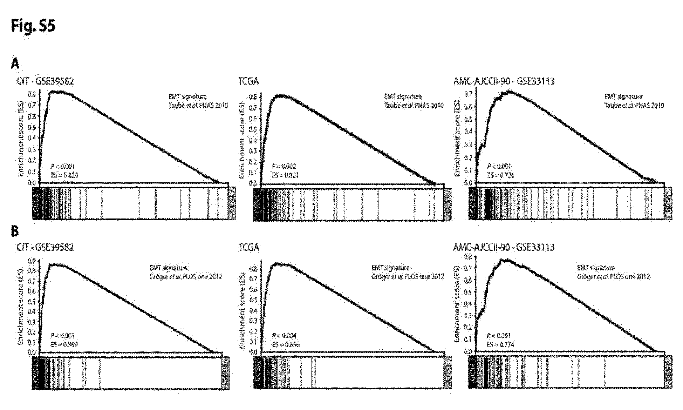

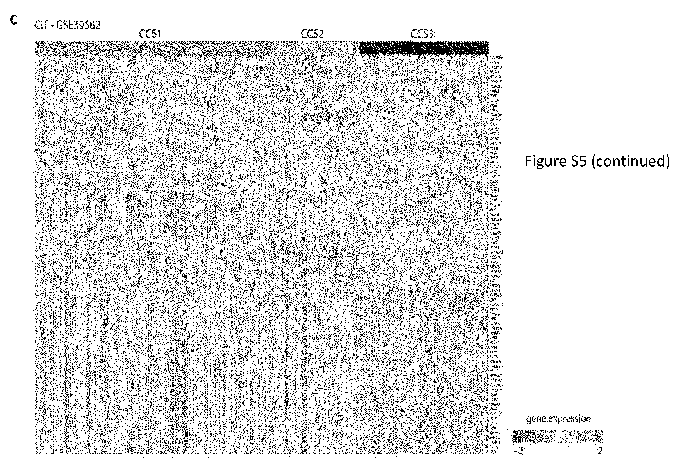

Figure 5 shows that CCS3 cell lines often display high miR-200 promoter methylation levels, which are instrumental for miR-200 expression. (A) Promoter methylation levels of miR-200 family members in CCS1 and CCS3 cell lines (P < 0.01; P-values are based on two-tailed Student's t-tests, displayed is the mean + SEM, n=21 for CCS1 and n=18 for CCS3). (B) Cell lines that display high miR-200 promoter methylation (> 10%) express low levels of the miR-200 family members (n=33 for methylation < 10% and n=6 for methylation > 10%; *P < 0.05, **P < 0.01; P-values are based on two-tailed Student's t-tests, displayed is the mean + SEM). (C) Decitabine (DAC) treatment of the HUTU-80 cell line reveals that a reduction in methylation results in re-expression of the miRs (n=3, P-values are based on two-tailed Student's t-tests, *P < 0.05, ***P < 0.001). (D) Overexpression of miR-200 family members in four different CCS3 cell lines. Expression levels of the most significantly downregulated genes in response to miR-200 overexpression are shown. Genes were sorted based on average log2FC (FC = fold change), indicated by the purple bar. (E) GSEA of miR-200 overexpressing cell lines confirms that these miRs significantly regulate the CCS3-specific gene expression program (left) and the EMT signature (right, Taube et al. 2010; ES = enrichment score).

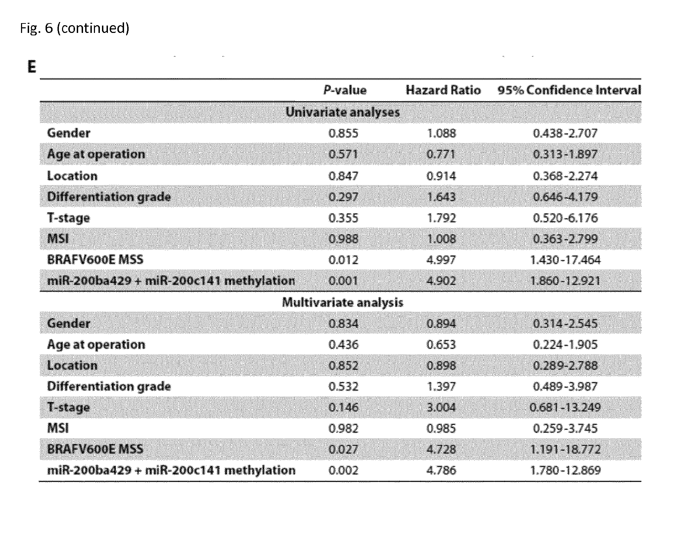

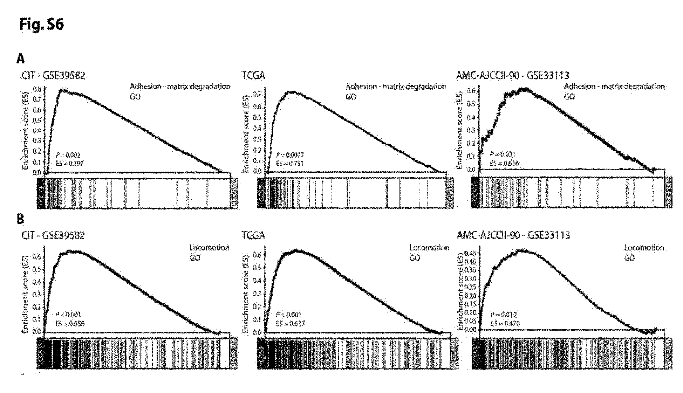

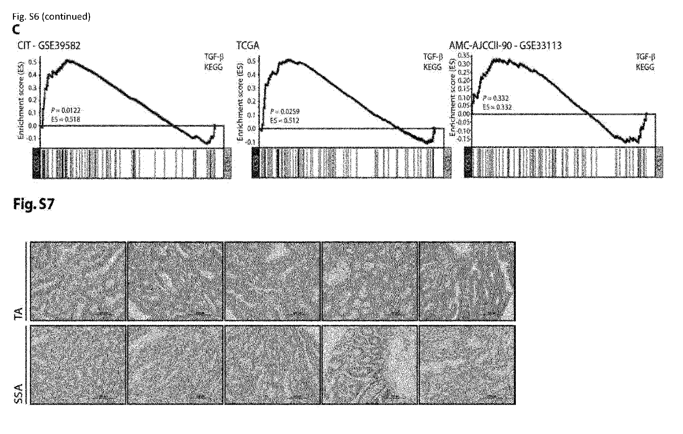

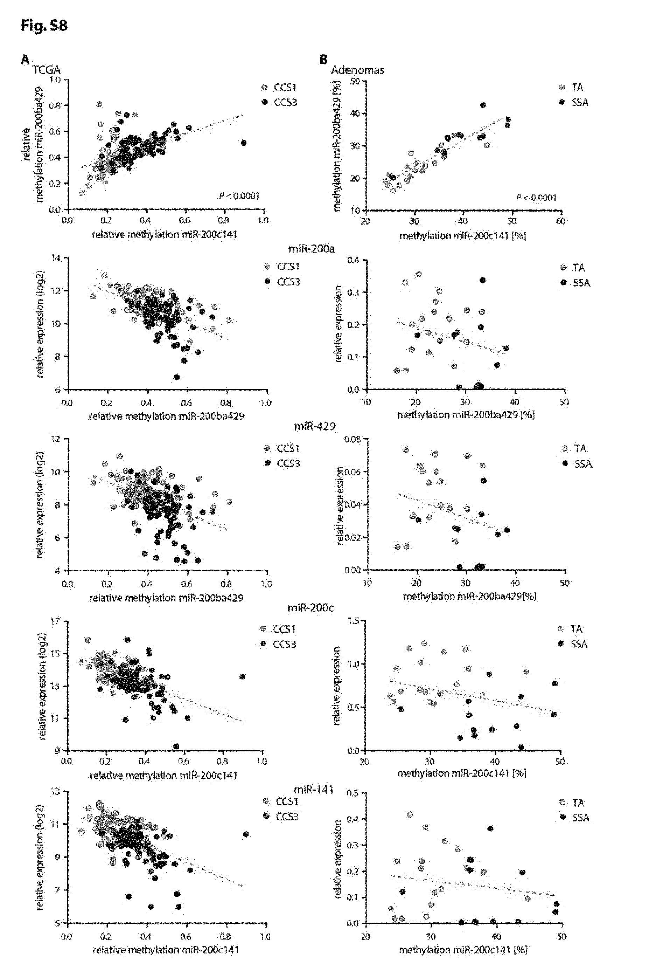

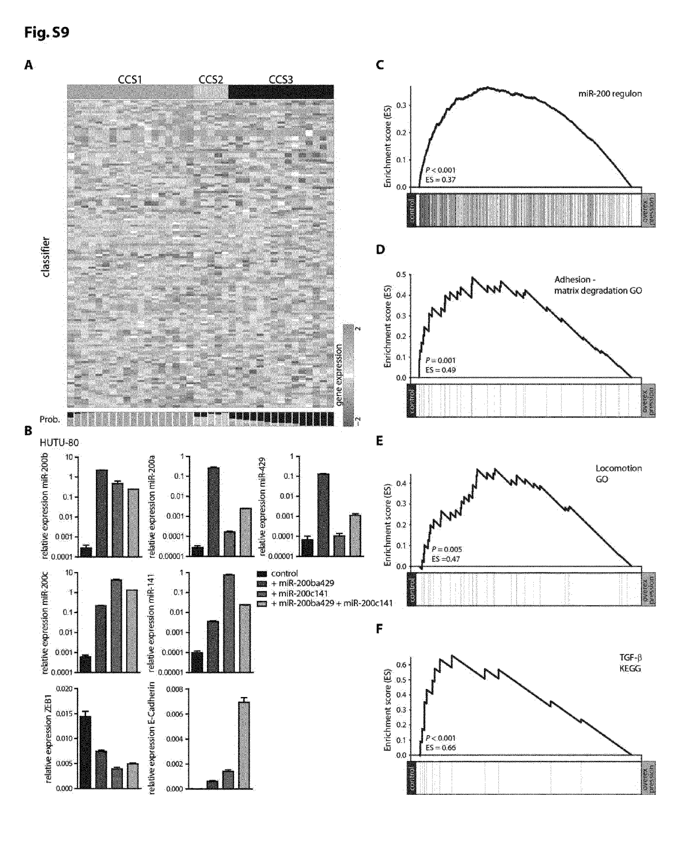

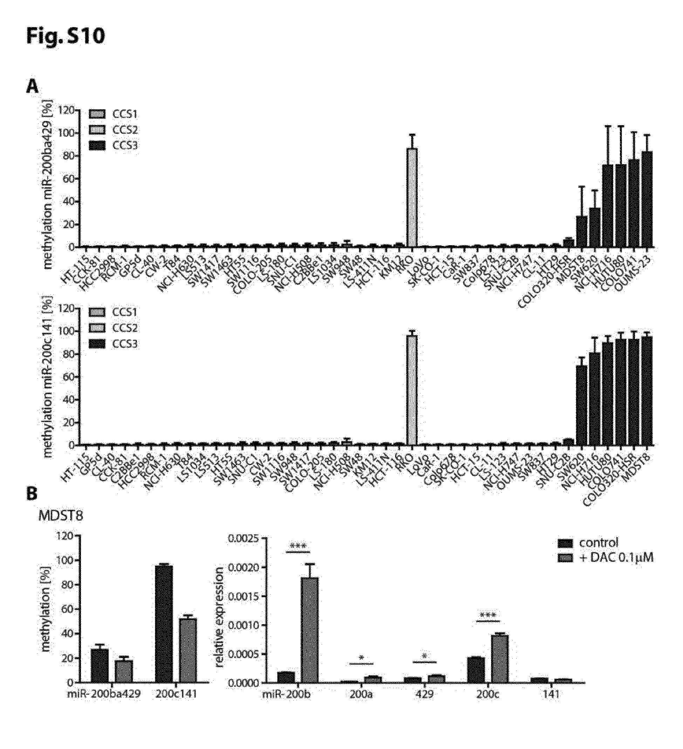

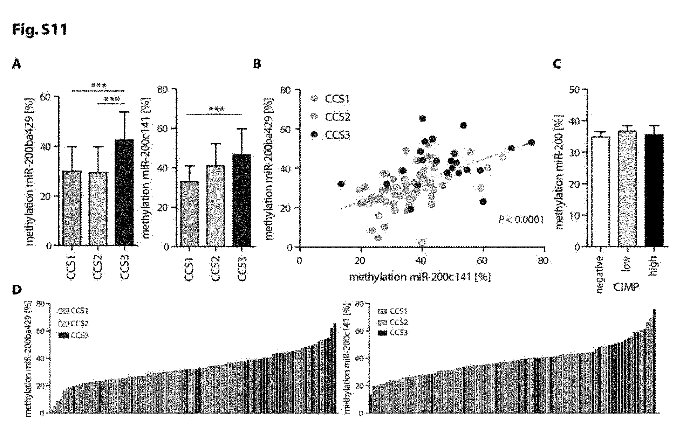

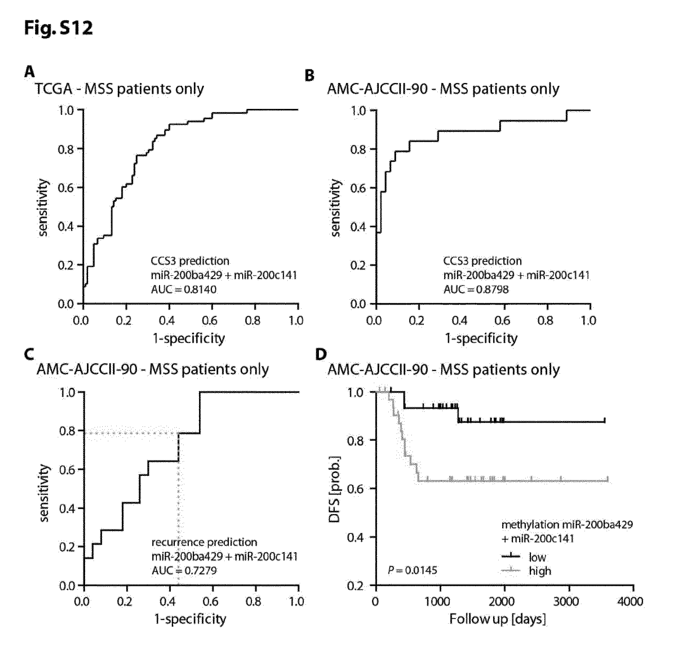

Figure 6 shows that miR-200 loci methylation is predictive of CCS3 affiliation and prognosis. (A and B) ROC curves showing the prediction of CCS3 affiliation in the TCGA (A) and AMC-AJCCII-90 (B) dataset using miR-200 loci methylation (AUC = area under the curve). (C) ROC curve displaying the survival prediction in the AMC-AJCCII-90 dataset. Orange lines indicate cut-off used for the KM curve shown in (D). (D) KM curve depicting DFS survival of the AMC-AJCCII-90 dataset separated based on the cut-off chosen using the ROC curve shown in (C). (E) Univariate (top) and multivariate (bottom) analyses of prognostic features in the AMC-AJCCII-90 set. Also in a multivariate analysis miR-200 promoter methylation remains an important independent prognostic factor (P = 0.002).
Figure S1 shows clinical and molecular information of the CIT, TCGA and AMC-AJCCII-90 datasets. (A) Characteristics of the datasets used in this study. TCGA 1 was used to generate the miR regulatory network and the TCGA 2 dataset for analysis of methylation as well as of methylation and expression correlations. (B) Flow of patients of the AMC-AJCCII-90 set through the study.
Figure S2 shows tumor location and stage of samples belonging to the CIT set. (A) The majority of CCS1 tumors is located distally, whereas most CCS2 tumors are found proximal. CCS3 tumors are equally distributed. (B) Stage 1+11 tumors are enriched in the CCS1 and CCS2 groups. More than 50% of the CCS3 samples are classified as stage III+IV tumors.
Figure S3 shows the master regulator networks of genes differentially expressed between CCS1 and CCS3 tumors. (A) Transcription factor (TF) regulatory network identifies no TF or cluster of TFs controlling large parts of subtype specific gene expression, albeit identifying some miR-200 controlled TFs. (B) Methylation of both miR-200 loci is identified as master regulator of the mesenchymal subtype by methylation mark master regulator network analysis as well as the methylation of other miRs that were also identified in the miR expression regulatory network. Moreover, several other candidate master regulators were identified, such as enhanced methylation of the loci that comprise the CDX2 gene and miR-192 in CCS3 tumors. Increased methylation of the Angiopoietin-like 2 (ANGPTL2) locus in CCS1 tumors was also predicted to be involved in regulation of the CCS1 - CCS3 distinction.
Figure S4 identifying miRNAs lowly expressed in CCS3 compared to CCS1 tumors ascertains the miR-200 family as driver of CCS3-related gene expression and the overrepresentation of EMT genes in the miRNA-associated regulons. (A) Identification of miRNAs based on differential expression between CCS1 and CCS3 tumors of the TCGA set (FC = fold change; P-values are based on two-tailed Student's t-tests and corrected for multiple hypothesis testing using the Benjamini-Hochberg method). (B) miR-200 members are significantly differentially expressed between CCS1 and CCS3 tumors (P-values are based on two-tailed Student's t-tests and corrected for multiple hypothesis testing using the Benjamini-Hochberg method; displayed is the mean + SEM). (C) Venn diagram depicting the overlap in putative targets of the miR-200 family members. (D) Shown is the regulon size of the miRNAs depicted in Fig. 2, i.e. number of genes that are regulated by the miRNA, and the significance of overrepresentation of EMT genes within the regulon of each miRNA. P-values are based on hypergeometric tests. (E) Displayed is the percentage of differentially expressed genes in CCS3 compared to CCS1 tumors that is predicted to be regulated by the indicated miRNA.
Figure S5 shows that CCS3 tumors express higher levels of genes associated with the EMT program. (A and B) GSEA of two EMT signatures (A Taube et al. 2010, B Gröger et al. 2012) in the CIT, TCGA and AMC-AJCCII-90 patient sets (ES = enrichment score). (C) Heatmap representing the median centered log2 gene expression levels of EMT-associated genes belonging to the Taube signature in patients of the CIT dataset (high expression: orange, low expression: blue). Rows indicate genes, columns represent patients. The subtypes are indicated in the bar above the heatmap.
Figure S6 shows that genes belonging to adhesion and matrix degradation, locomotion and TGF- signaling signatures are enriched in CCS3 tumors. GSEA of indicated gene sets (A) adhesion - matrix degradation (GO term), (B) locomotion (GO term), (C) TGF- pathway (KEGG pathway) in the CIT, TCGA and AMC-AJCCII-90 patient sets (ES = enrichment score). In general all gene sets are significantly enriched in the CCS3 subtype (left) in all patient series.
Figure S7 shows a representative histology of TA and SSA polyps.
Figure S8 shows that methylation of the miR-200 loci is highly correlated and is correlated with the expression of the respective miRNAs. The methylation of the miR-200c141 and miR-200ba429 loci in the TCGA dataset (A) and the adenoma dataset (B) are highly correlated. Additionally, methylation of the miR-200ba429 locus and of the miR-200c141 locus correlates with the expression of miR-200a and miR-429, miR-200c and miR-141, respectively.
Figure S9 shows that cell lines can be classified in the three CCSs and overexpression of miR-200 family members in CCS3 lines suppresses the expression of genes belonging to the miR-200 regulon and signatures associated with adhesion and matrix degradation, locomotion and TGF-signaling. (A) Heatmap of the Sanger cell line panel. 38 cell lines have been classified according to the CCS classification. Rows indicate genes of the 146-gene classifier, columns represent cell lines. The subtypes are indicated in the bar above the heatmap. The posterior probability of belonging to the subtype is shown in the bar below the heatmap. (B) Validation of miR-200 overexpression in the HUTU-80 cell line based on miR-200 member expression (log10 relative expression) and the expression of two target genes, ZEB1 and E-Cadherin. (C-F) GSEA of (C) the miR-200 regulon, (D) adhesion - matrix degradation (GO term), (E) locomotion (GO term), (F) TGF-beta pathway (KEGG pathway) signatures in control (left) vs. miR-200 overexpressing (right) cell lines (ES = enrichment score).
Figure S10 shows that CCS3 cell lines often present pronounced methylation of the miR-200 promoter regions which is instrumental for repressing miR-200 expression. (A) Analysis of miR-200 promoter methylation in a panel of 44 cell lines. Cell lines classified in the mesenchymal CCS3 subtype often present with pronounced methylation of these promoter regions. (B) Decitabine (DAC) treatment of the MDST8 cell line reveals that a modest reduction in methylation results in pronounced re-expression of the miRs (n=3, P-values are based on two-tailed Student's t-tests, *P < 0.05, ***P < 0.001).
Figure S11 shows that stage II CCS3 tumors display higher levels of miR-200 promoter methylation. (A) The methylation of the miR-200ba429 and miR-200c141 promoter regions was analyzed for 89 patients of the AMC-AJCCII-90 set and show significantly higher levels in tumors of the CCS3 class (P < 0.001; P-value is based on two-tailed Student's t-test). (B) The methylation of the miR-200 promoter regions is highly correlated, (C) but is not influenced by the CIMP status of the tumor. The average methylation of both miR-200 promoters taken together is shown. (D) Depicted is the methylation of the miR-200 promoters of 89 patients of the stage II AMC-AJCCII-90 dataset for each patient individually. CCS3 tumor samples are enriched in the quarter displaying the highest methylation.
Figure S12 shows that miR-200 promoter methylation is predictive of CCS3 affiliation and prognosis in MSS patients. (A and B) ROC curves showing the prediction of CCS3 affiliation in the TCGA (A) and AMC-AJCCII-90 (B) dataset using miR-200 loci methylation in MSS patients only. (C) ROC curve displaying the survival prediction in the AMC-AJCCII-90 dataset in MSS patients only. Orange lines indicate cut-off used for the KM curve shown in (D). (D) KM curve depicting DFS survival of the AMC-AJCCII-90 dataset separated based on the cut-off chosen using the ROC curve shown in (C).
Figure R3 shows the analysis of miR-200 methylation in primary cancers, matching cell lines and xenografts.
Figure R10 shows a scatter plot highlighting the significantly differentially expressed miRs between CCS1 and CCS3. X-axis indicates the difference in methylation between CCS1 and CCS3, and y-axis represents the absolute difference in expression. A modest inverse correlation was detected suggesting that CCS3 tumors display higher methylation of the miR loci that are lower expressed in this subtype.

### DETAILED DESCRIPTION OF THE INVENTION

### Definitions

Changes in cell phenotype between epithelial and mesenchymal states, defined as epithelial-mesenchymal (EMT) and mesenchymal-epithelial (MET) transitions, have key roles in embryonic development, and their importance in the pathogenesis of cancer and other human diseases is recognized (Polyak et al., 2009, Nature Rev., 272:265-73; Baum et al., 2008, Semin. Cell Dev. Biol. 9:294-308; Hugo et al., 2007, /. Cell Physiol. 273:374-83).

The term "EMT" refers to a complex molecular and cellular program by which epithelial cells shed their differentiated characteristics, including cell-cell adhesion, planar and apical-basal polarity, and lack of motility, and acquire instead mesenchymal cell-like features, including motility, invasiveness and a heightened resistance to apoptosis. Thus, similar to embryonic development, both EMT and MET seem to have crucial roles in the tumorigenic process. In particular, EMT has been found to contribute to invasion, metastatic dissemination and acquisition of therapeutic resistance. In contrast, MET-the reversal of EMT- seems to occur following cancer dissemination and the subsequent formation of distant metastases (Polyak et al., 2009, Nature Rev. 272:265-73). Importantly, initiation of the EMT program has been associated with poor clinical outcome in multiple tumor types (Sabbah et al., 2008, Drug Resist. Updat. 11 : 123-51), most likely because of the aggressive cell-biological traits that this program confers on carcinoma cells within primary tumors.

The term "colorectal cancer patient" as used herein refers to a patient (also referred to as subject) suffering from colon or rectal cancer, a subject suspected of having colorectal cancer, identified as having an increased risk for colorectal cancer, treated for colorectal cancer, in remission for colorectal cancer, being monitored for recurrence of colorectal cancer or a subject who has been treated for colorectal cancer. The patient could already be diagnosed as having colorectal cancer, and provided methods may be useful in determining the stage of disease progression, the type of tumor and the choice of therapy. In a preferred embodiment of any method according to the invention, said patient is suffering from colorectal cancer in stage 0, I, II, III or IV. In a highly preferred embodiment, said patient has been treated for colorectal cancer, preferably not longer than 10, 9, 8, 7, 6, 5 years ago. The colorectal patient may be an animal or human being. In preferred embodiments of the invention, the patient is a human being.

The term "suffering from" when used to describe a patient and in reference to a disease, is used herein to describe patients who have been diagnosed as having the disease, and/or are experiencing symptoms related to the disease. Thus, a patient who is diagnosed with colorectal cancer but not experiencing symptoms related to the cancer is "suffering from" colorectal cancer.

As used herein, the term "pre-cancer" or "pre-malignant cancer" refers to conditions which include the presence of cells that exhibit mild to severe dysplasia, such as adenoma. Such pre-malignant cancers include adenomatous polyps (adenomas) and polyposis syndromes. In preferred embodiments, such conditions comprise sessile serrated adenomas.

The term "colorectal cancer" as used herein, also called colon cancer or bowel cancer, is defined to include cancerous growths in the colon, rectum and appendix.

The term "susceptible" is used herein to mean having an increased risk for and/or a propensity for something, i.e. a disease such as cancer. The term takes into account that an individual "susceptible" for a disease may never be diagnosed with the disease.

As used herein, the terms "stage I cancer," "stage II cancer," "stage III cancer," and "stage IV" refer to the TNM staging classification for cancer. Stage I cancer typically identifies that the primary tumor is limited to the organ of origin. Stage II intends that the tumor has spread through the muscle wall of the colon. Stage III intends that the tumor has spread to lymph nodes. Stage IV intends that the primary tumor has spread to other organs.

The term "tumor relapse" as used herein refers to colorectal cancer that comes back again after treatment, either in the colon, or in some other part of the body.

The term "biological sample" as used herein refers to any sample from a colorectal cancer patient for diagnostic, prognostic, or personalized medicinal uses and may be obtained from surgical samples, such as biopsies or fine needle aspirates, from paraffin-embedded tissues, from frozen tumor tissue samples, from fresh tumor tissue samples, from a fresh or frozen body fluid. Most preferably the sample contains cells derived from colon or colorectal tissue or nucleic acids from such cells. However, any other suitable biological samples (e.g. bodily fluids such as blood, stool, etc...) in which the methylation status of a gene of interest can be determined are included within the scope of the invention. In certain embodiments of the invention, the biological sample is processed or treated before being used in accordance with the inventive methods. For example, the sample may be processed such that the sample contains mostly nucleic acids such as DNA and/or RNA. In a preferred embodiment, said biological sample is a paraffin-embedded tissue.

By "methylation status" is meant the level of methylation of cytosine residues (found in CpG pairs) in the gene of interest. When used in reference to a CpG site, the methylation status may be methylated or unmethylated. When used in reference to a CpG island or to any stretch of residues, the methylation status refers to the level of methylation, which is the relative or absolute concentration of methylated C at the particular CpG island or stretch of residues in a biological sample. Methylation of a CpG site or island at a promoter usually prevents expression of the gene. The sites or islands can also surround the 5' region of the coding region of the gene as well as the 3' region of the coding region. Thus, CpG sites or islands can be found in multiple regions of a nucleic acid sequence including upstream of coding sequences in a regulatory region including a promoter region, in the coding regions (e.g., exons), downstream of coding regions in, for example, enhancer regions, and in introns. All of these regions can be assessed to determine their methylation status, as appropriate. The levels of methylation of the gene of interest are determined by any suitable means in order to reflect whether the gene is likely to be downregulated or not.

The term "expression level" as used herein refers to a determined level of gene expression.

The term "hypermethylation" as used herein refers to the average methylation status corresponding to an increased presence of 5-mCyt at one or a plurality of CpG dinucleotides within a DNA sequence of a test DNA sample, relative to the amount of 5-mCyt found at corresponding CpG dinucleotides within a normal control DNA sample.

The term "under expression" as used herein refers to a lesser expression level of a gene in a biological sample, compared to expression in a control or reference sample. In preferred embodiments, under expression is defined as a decrease in expression level of at least twofold compared to the expression level value in a reference sample.

The phrase "corresponding to" when used to describe positions or sites within nucleotide sequences, is used herein as it is understood in the art. As is well known in the art, two or more nucleotide sequences can be aligned using standard bioinformatic tools, including programs such as BLAST, ClustalX, Sequencher, and etc. Even though the two or more sequences may not match exactly and/or do not have the same length, an alignment of the sequences can still be performed and, if desirable, a "consensus" sequence generated. Indeed, programs and algorithms used for alignments typically tolerate definable levels of differences, including insertions, deletions, inversions, polymorphisms, point mutations, etc. Such alignments can aid in the determination of which positions in one nucleotide sequence correspond to which positions in other nucleotide sequences.

The abbreviation "CpG" is used herein to refer to a dinucleotide comprised of a cytosine nucleotide (deoxycytidine) linked via a phosphate group to a guanine nucleotide (deoxyguanosine) through linkages to the 5' position of the deoxycytidine and the 3' position of the deoxyguanosine. The cytosine in this dinucleotide is said to be in the "5' position" of the dinucleotide, and the guanine is said to be in the "3' position" of the dinucleotide. As is understood by one of ordinary skill in the art, the abbreviation "CpG" also refers to modified dinucleotides similar, so long as the 5' nucleotide is still identifiable as deoxycytidine and the 3' nucleotide is still identifiable as deoxyguanosine. For example, a deoxycytidine-deoxyguanosine dinucleotide in which the cytosine ring is methylated at the 5 position is still considered a CpG dinucleotide, and may be referred to as a methylated CpG or abbreviated as 5mCpG. As used herein, the abbreviation CpG can also refer to a CpG site, defined below.

The term "CpG island" refers to a contiguous region of genomic DNA that satisfies the criteria of (1) having a frequency of CpG dinucleotides corresponding to an "Observed/Expected Ratio" >0.6, and (2) having a "GC Content" >0.5. CpG islands are typically, but not always, between about 0.2 to about 1 kb in length.

The term "Observed/Expected Ratio" ("O/E Ratio") refers to the frequency of CpG dinucleotides within a particular DNA sequence, and corresponds to the [number of CpG sites/(number of C bases X number of G bases)] X band length for each fragment.

The term "CpG site" is used herein to refer to a position within a region of DNA corresponding to a position where a CpG dinucleotide is found in a reference sequence. One of ordinary skill in the art will understand the term CpG site to encompass the location in the region of DNA where a CpG dinucleotide is typically found, whether or not the dinucleotide at that position is a CpG dinucleotide in a particular DNA molecule. For examples, the DNA sequence of a gene may typically contain a CpG dinucleotide at a particular position, but may contain other dinucleotides at the corresponding position in mutant versions, polymorphic variants, or other variations of the gene. Some mutations such as single base substitutions may alter the identity of the dinucleotide to be something other than CpG. Other mutations such as insertions or deletions may alter the position of the CpG dinucleotide typically found at a particular site. In cases such as these, the term CpG site is understood by one of ordinary skill in the art to encompass the site corresponding to the position where a CpG dinucleotide is typically found, for example, in a wild type version of the DNA. Similarly, the term CpG site also encompasses the corresponding site in a nucleic acid that has been modified experimentally, for example by labeling, methylation, demethylation, deamination (including conversion of a cytosine to uracil by a chemical such as sodium bisulfite), etc.

The term "prognosticating" as used herein means predicting or identifying the clinical outcome group that a subject belongs to according to the subject's similarity to a control group or control profile.

The term "diagnosing" means judging, predicting, assessing and/or evaluating as well as identifying and characterizing, including screening, whether a person is susceptible of or suffers from colorectal cancer or a pre malignant form thereof.

The term "Prognosis" is defined to include an assessment or prediction of the probable course, outcome, recovery or survival from a disease. Most physicians give a prognosis based on statistics of how a disease acts in studies on the general population. Prognosis can vary with colorectal cancer depending on several factors, such as the stage of disease at diagnosis, type of cancer, and even gender.

The term "predicting the response", as used herein refers to the determination of the likelihood that the patient will respond either favourably or unfavourably to a given therapy. Especially, the term "prediction", as used herein, relates to an individual assessment of any parameter that can be useful in determining the evolution of a patient. As will be understood by those skilled in the art, the prediction of the clinical response to the treatment with a demethylating agent, although preferred to be, need not be correct for 100% of the colorectal cancer patients to be diagnosed or evaluated. The term, however, requires that a statistically significant portion of colorectal cancer patients can be identified as having an increased probability of having a positive response. Whether a subject is statistically significant can be determined without further ado by the person skilled in the art using various well known statistic evaluation tools, e.g., determination of confidence intervals, p-value determination, Student's t-test, Mann- Whitney test, etc. Details are found in Dowdy and Wearden, Statistics for Research, John Wiley & Sons, New York 1983. Preferred confidence intervals are at least 50%, at least 60%, at least 70%, at least 80%, at least 90% at least 95%. The p- values are, preferably, 0.2, 0.1 or 0.05.

The term "clinical response", as used herein, refers to the response of the colorectal cancer patient to a therapy with a demethylating agent or other anti-tumor therapy. Standard criteria (Miller, et al. Cancer, 1981; 47:207-14) that can be used herewith to evaluate the response to a demethylating agent include response, stabilization and progression. It can be a complete response (or complete remission), which is the disappearance of all detectable malignant disease or a partial response, which is defined as approximately >50%> decrease in the sum of products of the largest perpendicular diameters of one or more lesions (tumor lesions), no new lesions and no progression of any lesion. Patients achieving complete or partial response are considered "responders", and all other patients are considered "non-responders".

The term "positive clinical response" as used herein refers to the outcome being either a complete or partial response.

The miR-200 family of microRNAs (also referred to as miRNAs or miRs) is composed by five distinct miRNAs, classified into two subfamilies according to the sequence homology in their seed region (sequence of the miRNA defining its target specificity corresponding to nucleotides 2-7). The first subfamily includes the miR-141 and miR-200a and the second one is composed by the miR-200b, miR-200c and miR-429. These miRNAs are located within two clusters on different genomic loci: miR-200b, miR-200a and miR-429 are located on chromosome 1 in the human genome (chromosome 4 in mouse) and miR-141 and miR-200c on human chromosome 12 (chromosome 6 in mouse).

The terms "miR-200ba429 promoter region" and "the miR-200c141 promoter region "as used herein preferably refers to the promotor regions described in Cell Cycle. 2013 Jul 15; 12(14): 2309-2320, Published online 2013 Jun 24. doi: 10.4161/cc.25405.

The term "biomarker" as used herein in various embodiments refers to a specific biochemical in the body that has a particular molecular feature to make it useful for diagnosing and measuring the progress of disease or the effects of treatment.

### Detailed description of certain embodiments of the invention

As miR-200 family members orchestrate subtype-specific gene expression and their epigenetic silencing could be installed at a premalignant stage and maintained throughout the progression to poor prognosis colon cancers, the inventors investigated whether the methylation of the miR-200 loci is a predictor of disease subtype and as a consequence identifies patients with poor prognosis. The current gold-standard classification method to identify CCSs consists of comprehensive gene expression arrays. Allocating patients based on miR-200 promoter methylation present a readily applicable and simple test. The inventors computed a binary logistic regression model using both miR-200 loci methylation levels as covariates. The resulting predicted probabilities (beta values) were used to calculate the receiver operator characteristics (ROC) curve, revealing that miR-200 loci methylation was able to identify CCS3-affiliation in the TCGA dataset (Fig. 6A). Next, the inventors validated the biomarkers in an independent patient series. Therefore the inventors determined the methylation of the miR-200 promoter regions in the AMC-AJCCII-90 dataset comprising only stage II colon cancer patients (fig. S1, A and B). Tumors of the CCS3 group showed a significantly higher methylation of the miR-200 loci compared to CCS1 tumors (fig. S11A). The methylation of these two loci was highly correlated (fig. S11B); however, the methylation levels were not influenced by the CIMP status of the tumor (fig. S11C), indicating that it is not a feature of tumors with overall higher DNA methylation, but that these regions are specifically methylated. DNA methylation in the patients displayed a gradient for both loci, in which CCS3 samples were enriched in the quarter displaying the highest DNA methylation (fig. S11D). Also in this dataset, the combined methylation of the miR-200 loci was able to predict CCS3 affiliation with high confidence (Fig. 6B).

Because detection of stage II colon cancer patients that harbor a poor prognosis is of utmost importance as this provides the opportunity to treat these patients with adjuvant therapy the inventors investigated if miR-200 methylation directly predicts patient prognosis. Disease-free survival (DFS) data is available for all patients of the AMC-AJCCII-90 set, in which disease recurrence largely represents detection of metastases either in the liver, the lung or the peritoneal cavity. miR-200 promoter methylation was able to identify patients developing recurrences using a Cox regression model (Fig. 6C). The inventors selected the x-beta value of 0.293 as cut-off, resulting in the prediction of recurrences with a sensitivity of 68.4% and a specificity of 75.7%. A Kaplan-Meier (KM) curve was generated making use of this cut-off and indeed patients containing highly methylated tumors displayed a significantly poorer DFS compared to patients whose tumors showed lower methylation of the miR-200 loci (Fig. 6D). To exclude that the miR-200-based stratification leads to a separation of MSI samples that are known to have a better prognosis, the above-described analyses were done using only MSS patients of the TCGA and AMC-AJCCII-90 datasets, resulting in similar outcomes (fig. S12).

Multivariate analyses confirmed that miR-200 promoter methylation is an independent prognostic factor (*P* = 0.002) in the AMC-AJCCII-90 dataset also when taking T-stage, differentiation grade, MSS/MSI status, and BRAF mutations into account (Fig. 6E). Hence, the inventors identified the miR-200 promoter methylation as a critical molecular determinant of the mesenchymal CCS, therefore strongly correlating with the patient group that is at high risk for developing recurrences.
Based on these results, the invention provides a method of diagnosing, prognosticating, predicting or detecting pre-cancer, colorectal cancer (CRC) tumor progression, or metastasis in a human subject comprising the steps of:
(a) determining in a biological sample from said human subject the methylation status of one or more genes selected from the group consisting of miR-200a, miR-200b, miR-200c, miR-141, and miR-429, and
(b) determining the methylation status of one or more of said genes,
(c) determining the risk of developing CRC, metastasis and/or tumor relapse based on said methylation status, wherein hypermethylation of one or more of said genes indicates an increased risk of developing CRC, metastasis and/or tumor relapse.

In preferred embodiments of the invention, the detected methylation status is hypermethylation, an increase in the methylation of cytosines in CpG sites. The increase can be about 1%, about 5%, about 10%, about 20%, about 25%, about 30%, about 35%, about 40%, about 45%, about 50%, about 60%, about 65%, about 70%, about 75%, about 80%, about 85%, about 90%, about 95%, or more than about 95% greater than the extent of methylation of cytosines typically expected or observed for the CpG site or sites being evaluated.

Any of variety of techniques to detect methylation status can be used in the practice of the methods described herein. The following descriptions provide some examples of such techniques, and are not intended to limit the types of techniques that can be used with such methods. As will be understood of one of ordinary skill in the art, variations to the described techniques can also be used in accordance with inventive methods described herein. In certain embodiments of the invention, two or more methods of detecting methylation status are used together or in combination. In certain embodiments of the invention, the technique or techniques used to detect methylation status is or are quantitative. For example, such methods may provide estimates of the percentage of DNA molecules in a sample that are methylated at one or more particular CpG sites.

In the present invention, any nucleic acid sample, in purified or nonpurified form, can be used, provided it contains or is suspected of containing, a nucleic acid sequence containing a target locus (e.g., CpG-containing nucleic acid). One nucleic acid region capable of being differentially methylated is a CpG site, a sequence of nucleic acid with an increased density relative to other nucleic acid regions of the dinucleotide CpG. The CpG doublet occurs in vertebrate DNA at only about 20% of the frequency that would be expected from the proportion of G*C base pairs. In certain regions, the density of CpG doublets reaches the predicted value; it is increased by ten-fold relative to the rest of the genome. CpG sites have an average G*C content of about 60%, compared with the 40% average in bulk DNA. The sites take the form of stretches of DNA typically about one to two kilobases long.

In many genes, the CpG sites begin just upstream of a promoter and extend downstream into the transcribed region. Methylation of a CpG site at a promoter usually suppresses expression of the gene. The sites can also surround the 5' region of the coding region of the gene as well as the 3' region of the coding region. Thus, CpG sites can be found in multiple regions of a nucleic acid sequence including upstream of coding sequences in a regulatory region including a promoter region, in the coding regions (e.g., exons), downstream of coding regions in, for example, enhancer regions, and in introns. In a preferred embodiment, the level of methylation in the promotor region is determined. In a highly preferred embodiment, methylation in the 110 basepairs including 6 CpGs in the miR-200ba429 promoter region and 140 basepairs including 11 CpGs in the miR-200c141 promoter region is determined.

In general, the CpG-containing nucleic acid is DNA. However, the method according to the present invention may employ, for example, samples that contain DNA, or DNA and RNA containing mRNA, wherein DNA or RNA may be single-stranded or double-stranded, or a DNA-RNA hybrid may be included in the sample.

A mixture of nucleic acids may also be used. The specific nucleic acid sequence to be detected may be a fraction of a larger molecule or can be present initially as a discrete molecule, so that the specific sequence constitutes the entire nucleic acid. It is not necessary that the sequence to be studied be present initially in a pure form; the nucleic acid may be a minor fraction of a complex mixture, such as contained in whole human DNA. Nucleic acids contained in a sample used for detection of methylated CpG sites may be extracted by a variety of techniques such as that described by Sambrook, et al. (Molecular Cloning: A Laboratory Manual, Cold Spring Harbor, N.Y., 1989).

In a preferred embodiment, methylation is determined using a bisulfite method. When genomic DNA is treated with bisulfite, cytosine in the 5'-CpG'-3 region remains intact, if it was methylated, but the cytosine changes to uracil, if it was unmethylated. Accordingly, based on the base sequence converted after bisulfite treatment, PCR primer sets corresponding to a region having the 5'-CpG-3' base sequence may be constructed. Herein, the constructed primer sets are two kinds of primer sets: a primer set corresponding to the methylated base sequence, and a primer set corresponding to the unmethylated base sequence. When genomic DNA is converted with bisulfite and then amplified by PCR using the above two kinds of primer sets, the PCR product is detected in the PCR mixture employing the primers corresponding to the methylated base sequence, if the genomic DNA was methylated, but the genomic DNA is detected in the PCR mixture employing the primers corresponding to the unmethylated, if the genomic DNA was unmethylated. This methylation can be quantitatively analyzed by agarose gel electrophoresis.

In another preferred embodiment, methylation is detected using real time methylation-specific PCR. Real-time methylation-specific PCR is a real-time measurement method modified from the methylation-specific PCR method and comprises treating genomic DNA with bisulfite, designing PCR primers corresponding to the methylated base sequence, and performing real-time PCR using the primers. Methods of detecting the methylation of the genomic DNA include two methods: a method of detection using a TaqMan probe complementary to the amplified base sequence; and a method of detection using SYBR green. Thus, the real-time methylation-specific PCR allows selective quantitative analysis of methylated DNA. Herein, a standard curve is plotted using an in vitro methylated DNA sample, and a gene containing no 5'-CpG-3' sequence in the base sequence is also amplified as a negative control group for standardization to quantitatively analyze the degree of methylation.

In another preferred embodiment, methylation is detected using pyrosequencing method. The pyrosequencing method is a quantitative real-time sequencing method modified from the bisulfite sequencing method. Similarly to bisulfite sequencing, genomic DNA is converted by bisulfite treatment, and then, PCR primers corresponding to a region containing no 5'-CpG-3' base sequence are constructed. Specifically, the genomic DNA is treated with bisulfite, amplified using the PCR primers, and then subjected to real-time base sequence analysis using a sequencing primer. The degree of methylation is expressed as a methylation index by analyzing the amounts of cytosine and thymine in the 5'-CpG-3' region.

In another preferred embodiment, methylation is determined using PCR Using Methylated DNA-specific binding protein, quantitative PCR, And DNA Chip Assay. When a protein binding specifically only to methylated DNA is mixed with DNA, the protein binds specifically only to the methylated DNA. Thus, either PCR using a methylation-specific binding protein or a DNA chip assay allows selective isolation of only methylated DNA. Genomic DNA is mixed with a methylation-specific binding protein, and then only methylated DNA was selectively isolated. The isolated DNA is amplified using PCR primers corresponding to the promoter region, and then methylation of the DNA is measured by agarose gel electrophoresis.

In addition, methylation of DNA can also be measured by a quantitative PCR method, and methylated DNA isolated with a methylated DNA-specific binding protein can be labeled with a fluorescent probe and hybridized to a DNA chip containing complementary probes, thereby measuring methylation of the DNA. Herein, the methylated DNA-specific binding protein may be, but not limited to, McrBt.

In another preferred embodiment, methylation is determined using Methylation-Sensitive Restriction Enzyme. Detection of differential methylation can be accomplished by bringing a nucleic acid sample into contact with a methylation-sensitive restriction endonuclease that cleaves only unmethylated CpG sites.

In a separate reaction, the sample is further brought into contact with an isoschizomer of the methylation-sensitive restriction enzyme that cleaves both methylated and unmethylated CpG-sites, thereby cleaving the methylated nucleic acid.

Specific primers are added to the nucleic acid sample, and the nucleic acid is amplified by any conventional method. The presence of an amplified product in the sample treated with the methylation-sensitive restriction enzyme but absence of an amplified product in the sample treated with the isoschizomer of the methylation-sensitive restriction enzyme indicates that methylation has occurred at the nucleic acid region assayed. However, the absence of an amplified product in the sample treated with the methylation-sensitive restriction enzyme together with the absence of an amplified product in the sample treated with the isoschizomer of the methylation-sensitive restriction enzyme indicates that no methylation has occurred at the nucleic acid region assayed.

As used herein, the term "methylation-sensitive restriction enzyme" refers to a restriction enzyme (e.g., SmaI) that includes CG as part of its recognition site and has activity when the C is methylated as compared to when the C is not methylated. Non-limiting examples of methylation-sensitive restriction enzymes include MspI, HpaII, BssHII, BstUI and NotI. Such enzymes can be used alone or in combination. Examples of other methylation-sensitive restriction enzymes include, but are not limited to SacII and EagI.

The isoschizomer of the methylation-sensitive restriction enzyme is a restriction enzyme that recognizes the same recognition site as the methylation-sensitive restriction enzyme but cleaves both methylated and unmethylated CGs. An example thereof includes MspI.

Primers of the present invention are designed to be "substantially" complementary to each strand of the locus to be amplified and include the appropriate G or C nucleotides as discussed above. This means that the primers must be sufficiently complementary to hybridize with their respective strands under polymerization reaction conditions. Primers of the present invention are used in the amplification process, which is an enzymatic chain reaction (e.g., PCR) in which that a target locus exponentially increases through a number of reaction steps. Typically, one primer is homologous with the negative (-) strand of the locus (antisense primer), and the other primer is homologous with the positive (+) strand (sense primer). After the primers have been annealed to denatured nucleic acid, the nucleic acid chain is extended by an enzyme such as DNA Polymerase I (Klenow), and reactants such as nucleotides, and, as a result, + and - strands containing the target locus sequence are newly synthesized. When the newly synthesized target locus is used as a template and subjected to repeated cycles of denaturing, primer annealing, and extension, exponential synthesis of the target locus sequence occurs. The resulting reaction product is a discrete nucleic acid duplex with termini corresponding to the ends of specific primers employed.

The amplification reaction is PCR which is commonly used in the art. However, alternative methods such as real-time PCR or linear amplification using isothermal enzyme may also be used. In addition, multiplex amplification reactions may also be used.

In another preferred embodiment, methylation is determined using a Bisulfite Sequencing Method. Such methods typically comprise the steps of: bringing a nucleic acid-containing sample into contact with an agent that modifies unmethylated cytosine; and amplifying the CpG-containing nucleic acid in the sample using CpG-specific oligonucleotide primers, wherein the oligonucleotide primers distinguish between modified methylated nucleic acid and non-methylated nucleic acid and detect the methylated nucleic acid. The amplification step is optional and desirable, but not essential. The method relies on the PCR reaction to distinguish between modified (e.g., chemically modified) methylated DNA and unmethylated DNA. Such methods are described in U.S. Pat. No. 5,786,146 relating to bisulfite sequencing for detection of methylated nucleic acid. In real-time embodiments, quantitation of (hyper)methylation may be on an absolute basis, or may be relative to a constitutively methylated DNA standard, or may be relative to an unmethylated DNA standard. Methylation status may be determined by using the ratio between the signal of the marker under investigation and the signal of a reference gene where methylation status is known (such as β-actin for example), or by using the ratio between the methylated marker and the sum of the methylated and the non-methylated marker. Alternatively, absolute copy number of the methylated marker gene can be determined.

Suitable controls may need to be incorporated in order to ensure the method chosen is working correctly and reliably. Suitable controls may include assessing the methylation status of a gene known to be methylated. This experiment acts as a positive control to ensure that false negative results are not obtained. The gene may be one which is known to be methylated in the sample under investigation or it may have been artificially methylated. In one embodiment, the gene of interest may be assessed in normal cells, following treatment with SssI methyltransferase, as a positive control. Additionally or alternatively, suitable negative controls may be employed with the methods of the invention. Here, suitable controls may include assessing the methylation status of a gene known to be unmethylated or a gene that has been artificially demethylated. This experiment acts as a negative control to ensure that false positive results are not obtained. In one embodiment, the gene of interest may be assessed in normal cells as a negative control, in particular if the gene is unmethylated in normal tissues.

In a preferred embodiment, diagnosis of CRC or a premalignant colorectal cancer is determined, or an increased risk of developing metastasis and/or tumor relapse is determined, if hypermethylation is determined in the miR-200ba429 promoter region and/or in the 140 basepairs including 11 CpGs in the miR-200c141. Preferably, at least two, preferably three, four or five genes of the group consisting of MiR-200a, miR-200b, miR-200c, miR-141, and miR-429 are hypermethylated. An advantage thereof is that if more of said genes are hypermethylated, the sensitivity of the method is greater.

In preferred embodiments, methylation of one or more genes of the MiR-200 family members is combined with the determination of other biomarkers indicative of colorectal cancer, preogression thereof, or a premalignant phase thereof, such as expression levels of certain genes associated with a colorectal pre-cancer, colorectal cancer progression and/or relapse or metastasis. For example, other gene sets associated with poor prognosis features such as matrix remodeling, locomotion and TGF-β signaling, were enriched in CCS3 tumors (fig. S6), all underlining the aggressiveness of malignancies belonging to the mesenchymal subtype. Therefore, in a preferred embodiment, determination of methylation of MiR-200 family members is combined with expression levels of genes involved matrix remodeling, locomotion and TGF-β signaling, ZEB 1 and/or E-Cadherin.

The inventors found that methylation of each locus significantly correlated with expression of the respective miR-200 family. In other preferred embodiment, methylation status of one or more MiR-200 family members is therefore combined with expression levels of one or more of these genes.

Various techniques for determining the expression level of a gene are known in the art and can be used in conjunction with the present invention.

Preferably, said colorectal cancer patient is suffering from colorectal cancer in stage I II or III. In another preferred embodiment, said patient is suffering from CRC in stage II and III. In another preferred embodiment, said patient is suspected from suffering from CRC.

Also at the precursor stage, both promoter regions were significantly higher methylated in SSAs and methylation of both regions was highly correlated (Fig. 4C and fig. S8B). Additionally, the expression of each miR-200 family member correlated with the methylation of its respective locus in adenomas (Fig. 4D and fig. S8B). Premalignant SSAs are therefore already endowed with the molecular circuitry that relieves the inhibitory constraint on the EMT program. Therefore, in preferred embodiment, the method of the invention is used to detect premalignant forms of CRC, including but not limited to SSA, or to prognosticate CRC.

In certain embodiments of the invention, the detected methylation status is determined in part or wholly on the basis of a comparison with a control. The control can be a value or set of values related to the extent and/or pattern of methylation in a control sample. In certain embodiments of the invention, such a value or values may be determined, for example, by calculations, using algorithms, and/or from previously acquired and/or archived data. In certain embodiments of the invention, the value or set of values for the control is derived from experiments performed on samples or using a subject. For example, control data can be derived from experiments on samples derived from comparable tissues or cells, such as normal tissue adjacent to tumors.

In certain embodiments of the invention, a control comprising DNA that is mostly or entirely demethylated, at one or more of the CpG sites being analyzed, is used. Such a control might be obtained, for example, from mutant tissues or cells lacking methyltransferase activity and/or from tissues or cells that have been chemically demethylated. For example, controls may be obtained from tissues or cells lacking activity of methyltransferases Dnmt1, Dnmt2, Dnmt3a, Dnmt3b, or combinations thereof. Agents such as 5-aza-2'-deoxycytidine may be used to chemically demethylate DNA.

In certain embodiments of the invention, a control comprising DNA that is mostly or entirely methylated, at one or more of the CpG sites being analyzed, is used. Such a control might be obtained, for example, from cells or tissues that are known or expected to be mostly or entirely methylated at the CpG site or sites of interest. Such a control could also be obtained by cells or tissues in which methylation levels have been altered and/or manipulated, for example, by overexpression of methyltransferases (such as enzymes Dnmt1, Dnmt2, Dnmt3a, Dnmt3b, any of the bacterial 5-CpG methyltransferases, or combinations thereof). In certain embodiments of the invention, samples used to obtain control values are processed and/or manipulated in the same manner as the samples being evaluated. In a preferred embodiment, said control comprises a colorectal cancer cell line with a known methylation status.

In some embodiments, negative control samples are generated by treating positive control samples with a demethylating agent, preferably 5-aza-2'-deoxycytidine.

A nucleic acid can contain a regulatory region which is a region of DNA that encodes information or controls transcription of the nucleic acid. Regulatory regions include at least one promoter. A "promoter" is a minimal sequence sufficient to direct transcription, and renders promoter-dependent gene expression controllable for cell-type specific, tissue-specific, or inducible by external signals or agents. Promoters may be located in the 5' or 3' region of the gene. The number of nucleic acids in all or part of promoter regions can be used to measure CG-site methylation. Moreover, it is generally recognized that methylation of the target gene promoter proceeds naturally from the outer boundary inward. Therefore, an early stage of cell conversion can be detected by analyzing methylation in these outer areas of the promoter region.

### Medical treatment using a demethylating agent

In another aspect, the invention provides a method of predicting the response in a colorectal cancer patient to treatment to colorectal cancer. The drug for the treatment is a demethylating agent. In preferred embodiments said demethylating agent is used in combination with another chemotherapy. The presence of hypermethylation indicates that the genes of the miR200 family of the invention are methylated to a higher extend, which enables intervention with a demethylating agent. Therefore, this presence indicates a more positive clinical response to said treatment, whereas the absence of methylation or a lower level of methylation compared to a control sample indicates an unsuccessful clinical response to the treatment. If a positive clinical response to treatment with a demethylating agent is determined, the patient is identified or selected for a treatment with said demethylating agent. In other cases, the patient is not selected for treatment with a demethylating agent, and one or more alternative drug or medical intervention may be more beneficial for the treatment of the colorectal cancer patient.

Therefore, the invention concerns a method of predicting the response in a colorectal cancer patient to treatment with a demethylating agent comprising providing a biological sample of said colorectal cancer patient, and determining in said biological sample the methylation status of at least one CpG in the miR-200ba429 promoter region and/or the miR-200c141 promoter region, and predicting a positive clinical response to said treatment, if hypermethylation is determined in at least one CpG in the miR-200ba429 promoter region and/or the miR-200c141 promoter region.

The demethylating agent may be any demethylating agent which is suitable for a medical treatment. Many demethylating agents are known in the art, including 5'aza-cytidine, 5-aza-2'-deoxycytidine (decitabine), zebularine [1-(β-D-ribofuranosyl(dihydro-pyrimidin-2-1)] and L-methionine.

Demethylating agents, according to the present invention, are preferably selected from the group consisting of 5'aza-cytidine, 5-aza-2'-deoxycytidine (decitabine), zebularine [1-(β-D-ribofuranosyl(dihydro-pyrimidin-2-1)], L-methionine, inhibitors of histone deacetylase (HDAC) such as, for instance, valproic acid or trichostatin A, apicidine, hydralazine, procainamide (pronestyl), antisense oligonucleotides directed against DNA methyltransferase messenger RNA, their admixtures and derivatives thereof. Preferably, the demethylating agents to be used in the present invention are 5'aza-cytidine, decitabine and zebularine.

The demethylating agent is preferably used in a pharmaceutically effective amount. The term "pharmaceutically effective amount" shall mean that amount of a drug or pharmaceutical agent that will elicit the biological or medical response of a tissue, system, animal or human that is being sought by a researcher or clinician. This amount can be a therapeutically effective amount. It is of course preferable that this therapeutically effective amount will achieve the goal of improvement in disease severity and the frequency of incidence over treatment of the agent by itself, and/or of amelioration of adverse side effects typically associated with alternative therapies.

The invention further provides a method for identifying and/or selecting a colorectal cancer patient with colorectal cancer or treated for colorectal cancer suitable for treatment with a demethylating agent comprising providing a biological sample of said colorectal cancer patient, and determining in said biological sample the methylation status of at least one CpG in the miR-200ba429 promoter region and/or the miR-200c141 promoter region, and identifying and/or selecting the colorectal cancer patient for treatment with said demethylated agent if hypermethylation is determined in a CpG in the miR-200ba429 promoter region and/or the miR-200c141 promoter region.

Further provided is a method for selecting a suitable treatment regimen for colorectal cancer in a colorectal cancer patient comprising providing a biological sample of said colorectal cancer patient, and determining in said biological sample the methylation status of at least one CpG in the miR-200ba429 promoter region and/or the miR-200c141 promoter region, and selecting said demethylating agent for treatment if hypermethylation is determined in a CpG in the miR-200ba429 promoter region and/or the miR-200c141 promoter region.

The invention therefore further provides a method of treating a patient suffering from colorectal cancer with a demethylating agent comprising providing a biological sample of said colorectal cancer patient, and determining in said biological sample the methylation status of at least one CpG in the miR-200ba429 promoter region and/or the miR-200c141 promoter region, and selecting said demethylating agent for treatment if hypermethylation is determined in a CpG in the miR-200ba429 promoter region and/or the miR-200c141 promoter region.

### Kits for the detection of methylation in colorectal cancer

Still another aspect of the invention is a kit for assessing methylation in a test sample. The kit comprises optionally a reagent that (a) modifies methylated cytosine residues but not non-methylated cytosine residues, or that (b); modifies non-methylated cytosine residues but not methylated cytosine residues. The kit also comprises a pair of oligonucleotide primers that specifically hybridizes under amplification conditions to a consecutive stretch of nucleic acids present in the miR-200ba429 promoter region and/or the miR-200c141 promoter region, following treatment with a reagent. Preferably, said consecutive stretch comprises 12, 15, 18, or 20 nucleotides. Preferably, said primers span 110 basepairs including 6 CpGs in the miR-200ba429 and/or 140 basepairs including 11 CpGs in the miR-200c141 promoter region.

Kits according to the present invention are assemblages of reagents for testing methylation. They are typically in a package which contains all elements, optionally including instructions. In certain embodiments of the invention, guidance is provided as to how to interpret the detected methylation status in order to make a diagnosis or other assessment in relation to cancer.

The package may be divided so that components are not mixed until desired. Components may be in different physical states. For example, some components may be lyophilized and some in aqueous solution. Some may be frozen. Individual components may be separately packaged within the kit. The kit may contain reagents, as described above for differentially modifying methylated and non-methylated cytosine residues. Typically the kit will contain oligonucleotide primers which specifically hybridize to regions within 1 kb of the transcription start sites of the genes belonging to the miRNA200 family. Typically the kit will contain both a forward and a reverse primer for a single gene. If there is a sufficient region of complementarity, e.g., 12, 15, 18, or 20 nucleotides, then the primer may also contain additional nucleotide residues or other chemical moieties that do not interfere with hybridization but may be useful for other manipulations. Exemplary of such other residues may be sites for restriction endonuclease cleavage, for ligand binding or for factor binding or linkers. Other moieties may include detectable labels or specific binding moieties, such as biotin. The oligonucleotide primers may or may not be such that they are specific for modified methylated residues. The kit may optionally contain oligonucleotide probes. The probes may be specific for sequences containing modified methylated residues or for sequences containing non-methylated residues. The kit may optionally contain reagents for modifying methylated cytosine residues. The kit may also contain components for performing amplification, such as a DNA polymerase and deoxyribonucleotides. Means of detection may also be provided in the kit, including detectable labels on primers or probes. Kits may also contain reagents for detecting gene expression for one of the markers of the present invention. Such reagents may include probes, primers, or antibodies, for example. In the case of enzymes or ligands, substrates or binding partners may be used to assess the presence of the marker.

In certain embodiments of the invention, a set of oligonucleotide primers is provided, each of which is designed to hybridize to sodium bisulfite-modified nucleic acids of in the miR-200ba429 or the miR-200c141 promoter region. For example, a kit may contain oligonucleotide primers designed to hybridize to sodium bisulfite-modified nucleic acids of a gene of interest as well as oligonucleotide primers designed to hybridize to sodium bisulfite -modified nucleic acids of the promotor region of interest.

In certain embodiments of the invention, the kit provides reagents and/or instructions for sodium bisulfite sequencing analysis of the miR-200ba429 or the miR-200c141 promoter region nucleic acids. Such reagents may include any of, for example, sodium bisulfite, buffers and solutions, spin columns for separation and/or purification of nucleic acids, reaction tubes, etc.

In certain embodiments of the invention, kits further comprise a control or reference sample that comprises DNA that is mostly or entirely demethylated. The DNA in the reference sample may be demethylated globally (e.g., at all or most CpG sites), in particular genes or regions of genes, or at one or more particular CpG sites. In certain embodiments of the invention, kits further comprise a control or reference sample that comprises DNA that is mostly or entirely methylated. The DNA in the reference sample may be methylated globally (e.g., at all or most CpG sites), in particular genes or regions of genes, or at one or more particular CpG sites.

The above disclosure generally describes the present invention. Unless defined otherwise, all technical and scientific terms used herein have the same meaning as those commonly understood by one of ordinary skill in the art to which this invention belongs. A more complete understanding can be obtained by reference to the following specific examples which are provided herein for purposes of illustration only, and are not intended to limit the scope of the invention.

### EXAMPLE SECTION

### Abstract:

Unsupervised classification of gene expression profiles has resulted in the identification of biologically and clinically distinct colon cancer subtypes (CCSs). The subtype that associates with poor clinical outcome displays a mesenchymal gene expression profile. No driver mutation has been identified for this category and patients are heterogeneous with regard to commonly used clinical markers. Here we report a regulatory network consisting of the miR-200 family members that tunes the majority of genes differentially expressed in the poor prognosis CCS, including genes involved in the epithelial-mesenchymal transition (EMT) process. Our data indicate that the epigenetic silencing of the miR-200 family by promoter methylation is identifying the mesenchymal CCS and is predictive of disease-free survival in this malignancy. We demonstrate that the molecular features of poor prognosis colon cancer - expression of EMT-associated genes and miR-200 promoter methylation - can already be installed at the premalignant stage, suggesting a highly malignant potential of specific colon cancer precursor lesions.

### Results

### Gene expression profiling identifies a heterogeneous group of poor prognosis colon cancers

Based on gene expression profiling, colon cancers can be divided into three main colon cancer subtypes (CCSs) using a 146-gene classifier (2). To validate this finding, the classifier was applied to a large independent dataset of 566 colon cancer patients (Cartes d'Identité des Tumeurs, CIT) of which gene expression data, as well as clinical annotation and molecular characterization is publicly available (fig. S1A) (6). In accordance with our previous findings the majority of patients was classified as CCS1 (51.2%), followed by 28.4% of patients in the CCS3 group and the smallest group being CCS2 (19.4%) (Fig. 1, A and B). As reported before, CCS1 associated with the well characterized chromosomal-instable (CIN) tumors, as 87.2% of the tumors identified as CCS1 were CIN+ and mainly left-sided, displaying *KRAS* and/or *TP53* mutations. This CCS1 group was almost devoid of microsatellite instable (MSI), CpG island methylator phenotype positive (CIMP+) or *BRAF*-mutated tumors (Fig. 1B and fig. S2A). In contrast, the CCS2 group contained a high level of MSI and CIMP+ right-sided tumors, therefore associating with the reported MSI/CIMP+ type of colon cancer (Fig. 1B and fig. S2A) (22-24). There was no clear association of CCS3 tumors with any of the above-mentioned features, rendering it a heterogeneous group of colon cancer patients with respect to molecular aberrations commonly occurring in this disease (Fig. 1B). The CCS1 and CCS2 subgroups were enriched in stage 1+11 tumors, whereas more than 50% of CCS3 tumors were classified as stage III+IV (Fig. 1B and fig. S2B). Additionally, we observed a significantly poorer prognosis for patients with CCS3 tumors (Fig. 1C). Hence, applying the gene classifier derived from a homogeneous set of stage II colon cancer patients to an independent dataset of 566 colon cancer samples comprising stage 0-IV tumors confirmed its clinical relevance by identifying a subtype with significantly decreased disease-free survival (DFS).

### A microRNA regulatory network accounts for differential gene expression between CCS1 and CCS3 tumors

Mutation status or molecular characteristics such as MSI do not allow the distinction between CCS 1 and CCS3 tumors, indicating that the driving force for the aggressive CCS3 subtype is more complex. To investigate which regulatory network is predominantly responsible for differences in gene expression between the subtypes we generated three master regulator networks comprising various levels of molecular regulation. In particular we studied 1. transcription factors (TFs) (fig. S3A), 2. methylation marks (fig. S3B), and 3. miRNAs (Fig. 2) as putative subtype specific master regulators. Network analyses were performed to identify which level of regulation was most stringently associated with the subtype-specific gene expression distinction in the TCGA dataset (fig. S1A). The miRNA network was found to be the most powerful determinant of subtype-specific regulation of gene expression and the largest regulatory element is predicted to be responsible for Panobinostat, % of the variation in gene expression (fig. S4E). The largest elements in the TF- and methylation mark-based master regulator networks account for approximately 11.9% and 13.8%, respectively. The dominant regulatory element in the miRNA network was generated by miRNAs belonging to the miR-200 family, namely miR-200a, miR-200b, miR-200c, miR-141, and miR-429, representing miRNAs whose expression levels are highly significantly different between CCS1 and CCS3 tumors (fig. S4B). The members of the miR-200 family are organized as polycistronic transcripts on chromosome 1 (miR-200ba429) and chromosome 12 (miR-200c141) (25). The network analysis predicted that these miRNAs target similar genes, suggesting that the miR-200 family members are functionally related. Indeed, analyzing the overlap of genes most strongly predicted (P < 0.01) to be regulated by one or the other miR-200 family member based on the master regulatory analysis confirmed that the vast majority of genes was regulated by at least 2 miRNAs and a large proportion, including the EMT TF zinc finger homeodomain enhancer-binding protein 1 (ZEB1), by all 5 miRNAs belonging to the miR-200 family (fig. S4C). Both the TF- and methylation mark-based master regulatory networks strongly relate to the miR-200 cluster. Firstly, we identified methylation of the miR-200 loci to be regulating a significant proportion of the subtype specific gene expression (fig. S3B), and secondly miR-200 associated TFs, including ZEB1, ZEB2 and TWIST2 (twist family bHLH transcription factor 2), were predicted to be TF master regulators albeit with a much lower number of downstream regulatory elements (fig. S3A).
Together, this points to a critical role of the miR-200 regulatory network in orchestrating subtype-specific gene expression. As reported before, expression of the miR-200 family associated with low expression of mesenchymal genes (26), thus the low abundance of miR-200 family members in CCS3 tumors could explain their mesenchymal phenotype.
Biologically this is of special interest, since the miR-200 family was shown to regulate the epithelial-mesenchymal transition (EMT) for example by modulating ZEB1 levels (16, *17,* 27). As the EMT has been linked to poor disease outcome, it may also be involved in the decreased survival of patients with CCS3 tumors (*28*, *29*). Highlighting the genes belonging to an experimentally-derived EMT signature in the inferred regulatory network revealed that many of the EMT-associated genes were indeed predicted to be regulated by the identified miRNAs (Fig. 2). Most of the EMT genes belonged to the miR-200 family cluster, and reversely, further analysis revealed that 11 miRNAs, including all miR-200 family members, function as master regulators of the EMT signature (fig. S4D) (30). Consistent with the idea that miR-200 is instrumental in orchestrating an EMT signature in CCS3, gene set enrichment analyses (GSEA) revealed that genes present in two independent EMT signatures were highly enriched in CCS3 tumors of three independent datasets (CIT (6), TCGA (24), AMC-AJCCII-90) (fig. S5, A and B) (*30-32*). The high expression of EMT-associated genes in CCS3 tumors also became apparent when looking at the expression of the genes belonging to an experimentally-derived EMT signature in the CIT patient series (fig. S5C) (*6*, *30*). Additionally, other gene sets associated with poor prognosis features such as matrix remodeling, locomotion and TGF-β signaling, were enriched in CCS3 tumors (fig. S6), all underlining the aggressiveness of malignancies belonging to the mesenchymal subtype.

### The miR-200 regulatory network is already active in precursor lesions

Gene expression data pointed to a strong overlap between CCS3 tumors and sessile serrated adenomas (SSAs), suggesting that a proportion of CCS3 tumors might originate from this distinct precursor lesion. Importantly, the higher expression of core EMT genes is already apparent at this early stage of tumorigenesis (2), suggesting that the regulatory network could already be installed at a premalignant stage. Applying the EMT signatures to gene expression data from 7 tubular adenomas (TAs) and 6 SSAs (2), confirmed the higher expression of EMT-associated genes in SSA polyps (Fig. 3A). Also on protein level, expression of the EMT-related transcription factor ZEB1 was detected in SSA epithelial cells, whereas the epithelium of TAs was completely devoid of ZEB1 (Fig. 3B).
Projecting the gene expression data of the adenoma set onto the network that was generated using the expression data from colon cancer samples (Fig. 2) revealed that most genes were highly expressed in SSA polyps, highlighting the similarity between SSAs and CCS3 tumors. Since the identified miRNAs were lowly expressed in CCS3 tumors and thus presumably also in SSAs, most gene products were again inversely correlated with the respective miRNA confirming the repressive nature of the members of the miR-200 family on EMT-associated genes in adenomas (Fig. 3C). To further investigate the role of the miR-200 family members at the premalignant stage, we generated a set of 18 TAs and 21 SSAs (fig. S7). Quantitative real-time PCR revealed that all members of the miR-200 family were significantly lower expressed in SSAs compared to TAs (Fig. 3D). We therefore demonstrate that a program that was thought to be responsible for aggressiveness of late stage disease is often already installed at a premalignant stage of colon cancer and is correlated with a decreased expression of the miR-200 family.

### Methylation of promoter regions underlies differential expression of miR-200 family members

As the expression level of miR-200 family members in SSA and TA polyps corresponds with the one observed in CCS3 and CCS1 tumors, respectively, we speculated that a metastable regulatory mechanism, such as an epigenetic mark, is installed in the precursor lesions and maintained throughout the adenoma-carcinoma sequence. Indeed, the methylation mark regulator network identified the methylation of the miR-200 loci as master regulator of subtype-specific gene expression (fig. S3B) and promoter methylation has been described to directly regulate expression of miR-200 family members (18-21). Both miR-200 loci displayed significantly higher methylation levels in CCS3 compared to CCS1 colon cancer samples from the TCGA set (Fig. 4A). A similar trend was observed for the loci of other miRs that are differentially expressed between the subtypes (fig. S4A), however this could not be directly explained by subtype-specific differences in DNA methyltransferase expression (data not shown). Thus, whether these methylation marks are installed by a common mechanism remains to be investigated.
The methylation of the two miR-200 loci was highly correlated, confirming the role of all miR-200 family members in regulating a common gene expression program (fig. S8A). Furthermore, methylation of each locus significantly correlated with expression of the respective miR-200 family member (Fig. 4B and fig. S8A), suggesting it to be decisive for the differential miR-200 expression. Also at the precursor stage, both promoter regions were significantly higher methylated in SSAs and methylation of both regions was highly correlated (Fig. 4C and fig. S8B). Additionally, the expression of each miR-200 family member correlated with the methylation of its respective locus in adenomas (Fig. 4D and fig. S8B). Premalignant SSAs are therefore already endowed with the molecular circuitry that relieves the inhibitory constraint on the EMT program.

### miR-200 family members are instrumental for subtype-associated gene expression

To functionally validate the above-described observations, we made use of a large cell line panel (n=44) that could be classified in the three CCSs (fig. S9A). Also in this *in vitro* culture system, cell lines allocated to the mesenchymal CCS3 subtype presented with pronounced methylation of the miR-200 loci (Fig. 5A and fig. S10A). The highest levels of miR-200 promoter methylation were detected in CCS3 cell lines that also displayed low levels of miR-200 expression (Fig. 5B). Using decitabine (DAC) the miR-200 loci were demethylated, resulting in upregulation of the miR-200 family members, indicating the methylation to be instrumental in controlling miR expression (Fig. 5C and fig. S10B).
To confirm the role of miR-200 family members in regulating subtype-specific gene expression, miR-200 family members were introduced in four CCS3 cell lines resulting in high levels of miR-200 family member expression, downregulation of ZEB1 and upregulation of E-Cadherin (fig. S9B). Gene expression profiles were derived from these lines and a large set of genes was identified to be downregulated upon miR-200 overexpression (Fig. 5D). GSEA revealed that genes that were putatively regulated by miR-200 family members, i.e. the miR-200 family regulon in the network analysis, were significantly enriched in the gene set that is regulated by miR-200 expression (fig. S9C). Furthermore, genes upregulated in CCS3 compared to CCS1 tumors were significantly inhibited in the miR-200 overexpressing cell lines (Fig. 5E). This is in line with the finding that members of the miR-200 family were predicted to regulate almost 70% of genes differentially expressed between CCS1 and CCS3 tumors (fig. S4E). Moreover, miR-200 overexpression in cell lines suppressed EMT-associated genes (Fig. 5E), and genes involved in matrix remodeling, locomotion and TGF-β signaling (fig. S9, D-F), processes active in CCS3 tumors (fig. S5 and fig. S6). In combination, these findings validate the notion that the miR-200 family represents an important determinant of subtype-specific gene expression and functional properties.

### Methylation of the miR-200 loci is a determinant of CCS affiliation and is predictive of disease-free survival

As miR-200 family members orchestrate subtype-specific gene expression and their epigenetic silencing could be installed at a premalignant stage and maintained throughout the progression to poor prognosis colon cancers, we speculated that the methylation of the miR-200 loci is a predictor of disease subtype and as a consequence identifies patients with poor prognosis. The current gold-standard classification method to identify CCSs consists of comprehensive gene expression arrays. Allocating patients based on miR-200 promoter methylation would present a readily applicable and simple test. We computed a binary logistic regression model using both miR-200 loci methylation levels as covariates. The resulting predicted probabilities (beta values) were used to calculate the receiver operator characteristics (ROC) curve, revealing that miR-200 loci methylation was indeed able to identify CCS3-affiliation in the TCGA dataset (Fig. 6A). Next, we sought to validate the utility of this test in an independent patient series. Therefore we determined the methylation of the miR-200 promoter regions in the AMC-AJCCII-90 dataset comprising only stage II colon cancer patients (fig. S1, A and B). Tumors of the CCS3 group showed a significantly higher methylation of the miR-200 loci compared to CCS1 tumors (fig. S11A). The methylation of these two loci was highly correlated (fig. S11B); however, the methylation levels were not influenced by the CIMP status of the tumor (fig. S11C), indicating that it is not a feature of tumors with overall higher DNA methylation, but that these regions are specifically methylated. DNA methylation in the patients displayed a gradient for both loci, in which CCS3 samples were enriched in the quarter displaying the highest DNA methylation (fig. S11D). Also in this dataset, the combined methylation of the miR-200 loci was able to predict CCS3 affiliation with high confidence (Fig. 6B). Because detection of stage II colon cancer patients that harbor a poor prognosis is of utmost importance as this provides the opportunity to treat these patients with adjuvant therapy we investigated if miR-200 methylation directly predicts patient prognosis.
Disease-free survival (DFS) data is available for all patients of the AMC-AJCCII-90 set, in which disease recurrence largely represents detection of metastases either in the liver, the lung or the peritoneal cavity. miR-200 promoter methylation was able to identify patients developing recurrences using a Cox regression model (Fig. 6C). We selected the x-beta value of 0.293 as cut-off, resulting in the prediction of recurrences with a sensitivity of 68.4% and a specificity of 75.7%. A Kaplan-Meier (KM) curve was generated making use of this cut-off and indeed patients containing highly methylated tumors displayed a significantly poorer DFS compared to patients whose tumors showed lower methylation of the miR-200 loci (Fig. 6D). To exclude that the miR-200-based stratification leads to a separation of MSI samples that are known to have a better prognosis, the above-described analyses were done using only MSS patients of the TCGA and AMC-AJCCII-90 datasets, resulting in similar outcomes (fig. S12).
Multivariate analyses confirmed that miR-200 promoter methylation is an independent prognostic factor (P = 0.002) in the AMC-AJCCII-90 dataset also when taking T-stage, differentiation grade, MSS/MSI status, and BRAF mutations into account (Fig. 6E). Hence, we identified the miR-200 promoter methylation as a critical molecular determinant of the mesenchymal CCS, therefore strongly correlating with the patient group that is at high risk for developing recurrences.

### Discussion

Gene expression profiling is a powerful tool to detect distinct patient subgroups and multiple classification methods have been used to categorize colon cancer patients. Although not resulting in identical subdivisions, a high degree of conformity can be detected (33). In all reported classification approaches a poor prognosis colon cancer entity characterized by a mesenchymal gene expression profile was identified (1-6). Yet, it is not clear whether there is a common underlying mechanism for differences in gene expression or distinct clinical outcome. Herein, making use of the TCGA dataset, several miRNAs were identified as regulators of subtype-specific gene expression. The miR-200 family was predicted to be responsible for controlling the expression of more than ∼70% of genes differentially expressed between CCS1 and CCS3 tumors, including EMT-associated genes. Indeed we show that the EMT process, which was implicated in poor disease outcome before (*28*, *29*), is active in CCS3 tumors based on gene expression profiling and our data point to the fact that the EMT program as well as the miRNA regulatory network is already installed in SSA polyps, a precursor lesion that is associated with CCS3 tumors. Thus, even though the EMT process is thought to occur late in tumor progression and to demark the time point when a localized tumor is able to spread to distant organs (34), we show that already at a premalignant stage in colon cancer development this program can be active. This points to the fact that early in colon cancer formation SSA lesions possess features allowing them to progress into aggressive and metastatic types of colon cancer, highlighting the need for early identification and thorough surveillance. Indeed, in a mouse model of pancreatic cancer, activation of EMT and subsequent dissemination of cells was observed before full malignant conversion (35). In a follow up study Rhim *et al.* detected pancreatic epithelial cells in the circulation of patients with premalignant lesions (36).
It remains unresolved what proportion of SSAs develops into CCS3 tumors and what fraction gives rise to other subtypes, in particular MSI CCS2 cancers. It is important to unravel specific adenoma-carcinoma sequences in depth in the future to be able to select the premalignant lesions developing to the poor prognosis CCS3 subtype. Our data stress the need to identify such tumors already at an early stage, as surgical removal of these malignancies might not be sufficient, consistent with the high incidence of recurrences and development of distant metastases in this subgroup. As we describe here, the methylation of the miR-200 promoter regions could serve as a tool to identify these poor prognosis colon cancer lesions early on. This epigenetic mark is prognostic in stage II colon cancers and we provide an easy and validated test to identify poor prognosis stage II patients that could be used to select individuals that are likely to benefit from more aggressive therapy. It remains to be examined whether high methylation levels could also be used as a marker to identify patients within this group that benefit from adjuvant therapy. This warrants further investigation in appropriate clinical studies. In this regard, the simplicity and highly robust nature of the miR-200 promoter methylation assay might be of great benefit to the field as this will allow for improved retrospective analysis of clinical studies and design of novel trials aiming to improve therapeutic outcome in these patients. A further interesting avenue of study is to determine how intra-tumor heterogeneity impacts on the ability of our assays to determine the subtype of an individual tumor. Taken together, our data demonstrate that the epigenetic modification that is the driver for a process associated with poor prognosis (EMT) is by itself predictive of disease outcome. This methylation mark can be detected using a simple and robust assay already early in the development of colon cancer and thus presents a tool to identify poor prognosis patients at early stages.

### Study design

The objective of this retrospective study was to identify molecular markers specific for the poor prognosis CCS3 that could explain the highly distinct biology of this subtype. The study consisted of a series of bioinformatical analyses as well as controlled laboratory experiments measuring multiple parameters such as miR-200 promoter methylation, mRNA and miRNA expression levels and protein expression. We used regulatory network inference to predict master regulators of genes differentially expressed between CCS1 and CCS3 in the publicly available TCGA dataset. To validate the role of miR-200 family members, miR-200 overexpressing cell lines were generated of which gene expression data was derived. Using GSEA we determined the effect of miR-200 overexpression on the EMT, matrix remodeling, locomotion, and TGF-β signaling processes, and confirmed the overrepresentation of associated genes in CCS3 tumor samples of three independent datasets comprising more than 900 patients in total. The samples collected at our institute were obtained as frozen tissues from the pathology department of the AMC, following appropriate patient consent according to the institute's guidelines (Medical Ethical Committee, AMC, Amsterdam). All analyses were performed blinded with respect to the subtype and disease-free survival data. Prior to the study, the reproducibility of the miR-200 promoter methylation assay was tested using control cell lines.

### Sample collection and CIMP analysis

Frozen tissues were collected from the pathology department (AMC), following appropriate patient consent according to the institute's guidelines (Medical Ethical Committee, AMC, Amsterdam).

Stage II colon cancer cases of the AMC-AJCCII-90 patient series were included from 1992 to 2005 and the last recorded follow-up was in 2012. Clinical endpoints include disease-free survival (DFS) after curative and radical surgery for colon cancer. Clinical or radiological apparent distant metastases (mainly liver and lung) and local recurrence were regarded as events. Samples containing a tumor cell percentage of > 70% and sufficient RNA quality (RIN > 7) were included in the microarray (GSE33113). The gDNA quality of one sample was not sufficient for determination of miR-200 promoter methylation and was thus excluded from the analysis.

Tubular adenomas (TAs) were obtained from familial adenomatous polyposis (FAP) patients carrying an APC mutation and therefore predictably progress through the CIN pathway to CCS1 tumors. Sessile serrated adenomas (SSAs) were collected from serrated polyposis syndrome (SPS) patients. We confirmed the nature of each polyp by H&E staining (fig. S7).

5x20 µm frozen tissue sections were cut using a cryotome and stored at -80°C until further use.

RNA was extracted using the miRNA easy micro kit (Qiagen) from 18 TA and 15 SSA polyps. In the detection of miR-200 family expression (Fig. 3D) we included 6 SSA polyps used for gene expression analysis (2).

Matching genomic DNA (gDNA) was extracted for all 18 TAs and for 13 SSAs using the High Pure PCR Template Preparation Kit (Roche).

2 µg of gDNA were subjected to bisulfite conversion using the EpiTect Bisulfite Kit (Qiagen) according to the manufacturer's instructions. TaqMan-based methylation specific quantitative real-time PCR (MethyLight) was used to determine the CIMP-status of the AMC-AJCCII-90 samples. 8 CIMP-specific markers (CACNA1G, IGF2, NEUROG1, RUNX3, SOCS1, MLH1, CRABP1, CDKN2A) and a methylation-independent normalization control (ALU) were included in the analysis. Primer/probe sequences and protocol are described previously (2, 37, 38). Markers with a PMR value >10 were considered positive for methylation; samples were defined as CIMP high (≥6 out of 8 CIMP markers have a PMR>10), low (1-5 out of 8 CIMP markers have a PMR>10) or negative (0 markers have a PMR>10).

### Colon cancer subtype (CCS) classification

For the CIT dataset, microarrays for all 566 patients (GSE39582) were first normalized and summarized using frozen robust multiarray analysis (fRMA) (39). Non-biological effects across batches were detected using principal component analysis and were corrected using ComBat (40). The expression profiles were median centered and then subjected to classification using the CCS classifier as previously described (2).

Two independent TCGA RNASeq datasets, based on Illumina GA and HiSeq platforms, were used for constructing a regulatory network and for correlation analysis, respectively. For the Illumina GA dataset, normalized RPKM (reads per kilobase per million mapped reads) profiles for 270 patients were publicly available in (24). For the Illumina HiSeq dataset, we obtained level-3 RSEM normalized count data for 332 patients from the TCGA data portal. For each dataset, the expression profiles were first log2-transformed and median centered. We next removed redundant gene replications by selecting those with the highest overall expression and substituted signature genes missing in the TCGA dataset with their most correlated genes in the AMC-AJCCII-90 dataset. Finally, the preprocessed expression profiles were subjected to our CCS classifier for subtype classification.

Using the same strategy, we also classified the Sanger colorectal cancer cell line panel (n=38, fig. S9A) based on the gene expression profiles obtained from ArrayExpress database (E-MTAB-783). For six cell lines in our panel no gene expression data was available and we used the classification from our original study (2).

Although the methylation changes reported here are somewhat subtle, the overall trend holds. We hypothesized that the tumor mesenchyme might obscure the actual tumor epithelial cell specific signal of miR-200 methylation as it has been reported before that miR-200 family loci are often methylated in tumor associated fibroblasts, but are for example not expected to be methylated in infiltrating lymphocytes. To investigate the tumor cell specific methylation of miR-200 loci we investigated this epigenetic mark in a large cell line panel (n=44, kind gift from the Sanger Institute) that has been classified into the various subtypes (see fig. S9A). We found that miR-200 methylation occurs predominantly in the cell lines representative of the mesenchymal subtype (see fig. S10A). Of note we observe that in case methylation is detected this occurs at high levels (>80-90%). In addition we have compared primary colon cancers with xenografts and cell lines that have been derived from these cancers (Fig. R3). We detected that in some cases the methylation signal found in the primary tumor was derived from the stroma, as corresponding cell lines did not display any methylation of the miR-200 loci. In cases of fairly high primary tumor methylation levels the epithelium contributed to this signal, as matching cell lines and xenografts displayed very high levels of tumor cell specific promoter methylation. Combined these analyses indicate that although the overall differences in methylation levels appear subtle this reflects a much more marked difference in epithelial cell specific methylation levels. Furthermore, we now show that relatively limited changes in methylation, as induced by low concentration treatment with the demethylating agent decitabine (DAC), result in a modest reduction in methylation of the miR-200 loci but marked upregulation of the miRs themselves (fig. S10B). In conclusion, although the absolute differences in methylation levels between the subtypes are limited albeit highly significant (Fig. 4), but that these differences are in fact large enough to be used to identify the subtypes (Fig. 6), and that they reflect much larger epithelial cell specific levels of differentiation.

### Inference of transcription factor regulatory network

From the total number of 1544 transcription factors (TFs) obtained from AnimalTFDB (41), we first selected 91 TFs significantly differentially expressed between CCS3 and CCS1 (|log2FC| > 1, FDR < 0.001). The same approach used for inference of the miRNA regulatory network was employed here to infer a network encoding regulatory relationships between TFs and target genes (fig. S3A).

### Inference of DNA methylation regulatory network

In total, 51 genes and 8 miRNAs were selected as regulators based on two criteria: (1) significant anticorrelation between their expression profiles and DNA methylation profiles (Pearson correlation coefficient < -0.5, P-value < 0.001); (2) significant differential DNA methylation between CCS3 and CCS1 samples (|log2FC| > log2(1.1), FDR < 0.001). 2262 genes significantly differentially expressed between CCS3 and CCS1 (|log2FC| > 0.5, FDR < 0.001) were selected as target genes of the total 59 regulators. Using the same approach for miRNA network inference, we predicted a network representing how DNA methylation regulates gene expression (fig. S3B).

### GSEA

Gene set enrichment analysis (GSEA) was performed for two EMT gene sets, adhesion and matrix-degradation, locomotion, TGF-, and miR-200 signatures (30-32). We took as phenotype log fold change between gene expression of TAs and SSAs, CCS1 and CCS3 samples, or control and miR-200 overexpressing cell lines. P-values indicating the significance of enrichment were estimated by 1000 permutations.

### Generation of the EMT heatmap

The expression of genes belonging to the Taube EMT set for samples of the CIT dataset was imported into MultiExperiment Viewer (http://www.tm4.org/mev.html) and genes were normalized by rows and median centered (6, 30). Depicted in fig. S5C is a hierarchical cluster of the gene tree using Pearson Correlation.

### Regulatory network inference

We employed a network-based approach to study the regulatory relationships between miRNAs and potential target genes. Gene expression data used here are log2-transformed RPKM profiles (n=270) in the TCGA Illumina GA dataset, while the miRNA data are log-transformed RPM (reads per million miRNA mapped, n=255) obtained from (24). Together, 249 patient samples have both miRNA and gene expression data. Specifically, we focussed on 30 miRNAs that are downregulated (fold change < 0.72, FDR < 0.001) in CCS3 compared to CCS1, and 1284 genes differentially expressed between CCS1 and CCS3 (|log2FC| > 1, FDR < 0.001). The miRNA expression data and gene expression data were standardized independently and merged together for network inference.

From the preprocessed data we first constructed a mutual information (MI) network, based on which we inferred a regulatory network by selecting significant interactions (FDR < 0.01) using a permutation analysis and removing unstable interactions (consensus score ≤ 0.95) by a bootstrap analysis. For better visualization, we applied the DPI (data processing inequality) criterion of the ARACNE algorithm (tolerance = 0) to the regulatory network (42). The filtered regulatory network with the vast majority of indirect interactions removed is presented in Fig. 2. All network analyses were performed using the RTN package available in bioconductor.

### Master regulator analysis

Having obtained a regulatory network, we performed master regulator analysis to identify master regulators of the EMT program. In detail, we tested the statistical significance of overrepresentation of EMT signature genes in each miRNA's regulon (target genes of the miRNA). The resulting P-values were corrected for multiple hypothesis testing using the Benjamini-Hochberg (BH) method. 11 miRNAs of top significance (BH-adjusted P-value < 0.05) were selected as master regulators (fig. S4D).

### TCGA data processing

Multiple TCGA datasets were used for analyzing the correlation between expression and promoter methylation of the miR-200 family members (fig. S1A; Fig. 4, A and B and fig. S8A). miRNA data are log2-transformed level-3 RPM profiles (n=250) based on Illumina HiSeq platform. DNA methylation data are level-3 beta values (n=339) indicating methylation levels based on Illumina Infinium HumanMethylation450 Array platform. Both datasets were downloaded from the TCGA data portal. Altogether 229 patient samples have miRNA expression and methylation profiles, as well as the above-mentioned mRNA expression profiles based on Illumina HiSeq platform. For each miR-200 family member, we took the median beta value over all annotated CpG sites as its promoter methylation level. The mean methylation levels over corresponding individual miR-200 family members were used for the miR-200ba429 and miR-200c141 clusters, respectively.

### Cell Line Culture and Treatment

We obtained 44 colorectal cancer cell lines as a kind gift from the Sanger Institute. The cell lines were cultured in D-MEM/F-12 medium with L-Glutamine, 15 mM HEPES (Invitrogen) supplemented with 8% FCS (Lonza) or in RPMI 1640 medium with L-Glutamine, 25 mM HEPES (Invitrogen) supplemented with 8% FCS (Lonza), 1% D-Glucose Solution Plus (Sigma), and 100 M Sodium Pyruvate (Life Technologies).

To induce demethylation, 50.000 cells were seeded per well in a 6-well plate in medium containing 0.1 M decitabine (DAC, 5-Aza-2'-deoxycytidine, Sigma) or the matching concentration of DMSO as control. DAC and DMSO were refreshed every 24 hours. After 96 hours, cells were collected and gDNA was extracted, bisulfite converted, and methylation was analyzed by pyrosequencing as described below. RNA was extracted using the NucleoSpin miRNA kit (Macherey-Nagel) and miRNA expression was analyzed as described below.

### miR-200 overexpression

HEK293T cells were transfected with either the human pre-microRNA expression construct lenti-miR-200b+200a+429 or the human pre-microRNA expression construct lenti-miR-200c+141 (Systems Biosciences) together with third generation lentiviral packaging plasmids according to the manufacturer's instructions. Four CCS3 cell lines with high miR-200 promoter methylation (HUTU-80, MDST8, NCI-H716, and SW620) were transduced with lentiviral supernatant and sorted on GFP-positive cells after 48 hours. 24 hours after sorting a fraction of the miR-200ba429 transduced cells was transduced with miR-200c141 lentivirus. For single- and double-transduced cells, RNA was extracted 7 days after transduction using the NucleoSpin miRNA kit (Macherey-Nagel) and miRNA and RNA expression levels were determined as described below. The overexpression system was validated by determining miR-200, ZEB1 and E-Cadherin expression levels in all four cell lines (HUTU-80 is shown as an example in fig. S9B).

### Microarray

Microarrays of miR-200 overexpressing cell lines were performed using the GeneTitanTM MC system from Affymetrix according to the standard protocols of the Cologne Center for Genomics (CCG), University of Cologne, Germany.

### Rank Product analysis and gene set enrichment analysis on gene expression profiles before and after miR-200 overexpression

Microarrays (n=16) for 4 different CCS3-like cell lines before and after overexpression of miR-200 members were first normalized and summarized using RMA (43). To identify differentially expressed genes, we employed one-class Rank Product analysis (44), using log2-fold changes of gene expression between each cell line treated with overexpression of miR-200 family member(s) and corresponding control. Expression levels of the most significantly downregulated genes (PFP < 0.01, log2FC < -0.5) in response to miR-200 overexpression in different cell lines are shown in Fig. 5D.

GSEA were performed to investigate whether overexpression of the miR-200 family members in cell lines suppresses expression of gene signatures associated with EMT, matrix-remodeling, cell migration, and TGF-β signaling pathways, regulons of miR-200 family members, as well as all genes significantly upregulated in CCS3 compared to CCS1 tumors. In the GSEA, we used as phenotype the ranks of the rank product of genes. We observed significant enrichment of expression of these signatures in the control (BH-adjusted P-value < 0.001), suggesting that compared to controls the tested pathways and miR-200 regulons are significantly inhibited by miR-200 overexpression.

### Quantitative real-time PCR miRNA

RNA was extracted using the miRNAeasy micro kit (Qiagen) for patient samples and the NucleoSpin miRNA kit (Macherey-Nagel) for cell lines. For miRNA expression analysis, 1 µg of total RNA was reverse transcribed to cDNA using the HiFlex buffer of the miScript II RT Kit (Qiagen). Quantitative real-time PCR (qRT PCR) was performed using the miScript SYBR Green PCR Kit (Qiagen) together with the following miScript Primer assays: Hs_RNU6-2_11 Hs_SNORA74A_11 Hs_miR-200b_3, Hs_miR-200a_1, Hs_miR-429_1, Hs_miR-200c_1, and Hs_miR-141_1 according to the manufacturer's instructions. Data shown was normalized to the expression of SNORA74A, normalization to RNU6-2 yielded comparable results.

### mRNA

To determine gene expression levels by qRT PCR, 1 g total RNA was reverse transcribed to cDNA using Superscript III following the manufacturer's protocol (Invitrogen). qRT PCR was performed using SYBR Green and a Roche Light Cycler 480 II in accordance with the manufacturer's instructions. All obtained values were normalized to the expression of GAPDH.

| | Forward primer | Reverse primer |
|---|---|---|
| GAPDH | AATCCCATCACCATCTTCCA(SEQ ID NO:7) | TGGACTCCACGACGTACTCA(SEQ ID NO:10) |
| ZEB1 | GCACAAGAAGAGCCACAAGTA (SEQ ID NO:8) | GCAAGACAAGTTCAAGGGTTC(SEQ ID NO:11) |
| E-Cadherin | TGGAGGAATTCTTGCTTTGC (SEQ ID NO:9) | CGCTCTCCTCCGAAGAAAC (SEQ ID NO:12) |

### miR-200 promoter methylation analysis

gDNA was extracted and bisulfite converted as described above. Methylation detection was carried out using the PyroMark PCR system (Qiagen). Primers were designed using the PyroMark Assay Design Software 2.0 (Qiagen) and span 110 basepairs including 6 CpGs in the miR-200ba429 promoter region and 140 basepairs including 11 CpGs in the miR-200c141 promoter region. PCR reactions and pyrosequencing were performed following the manufacturer's instructions.

Depicted is the average methylation of all 6 or 11 CpGs taken together.

| | miR-200ba429 | miR-200c141 |
|---|---|---|
| Forward primer | GGTTTGAATTGATTTTTTGTG TTAGG (SEQ ID NO:1) | ATTGTAGAGGGGGGATGAG (SEQ ID NO:4) |
| Reverse primer | CCTCAACCAAAATCAAACCT CA (SEQ ID NO:2) | CCAAATTACAATCCAAACAAACC (SEQ ID NO:5) |
| Sequencing primer | ATTTTTTGTGTTAGGGTTT (SEQ ID NO:3) | GATGAGGGTGGGTAA (SEQ ID NO:6) |

### Immunohistochemistry

4 µm sections of formalin fixed paraffin embedded tissues were used for immunohistochemistry. After rehydration, antigen retrieval was performed using an EDTA-based buffer at pH9.0 (Vector Laboratories) for 20 minutes at 98C. Endogenous peroxidase was blocked with 3% H2O2 (VWR) in PBS followed by incubation in ultraV block (Immunologic) for 10 minutes at RT. Sections were incubated in primary antibody dilution at 4C over night (anti-ZEB 1 (1:400, Sigma HPA027524)). Subsequently, post-antibody blocking (Immunologic) was added for 20 minutes at RT followed by Poly-HRP-Anti-mouse/rabbit/rat IgG (Immunologic) for 30 minutes at RT. Sections were incubated in Bright DAB solution (Immunologic), rinsed in dH2O and counterstained using hematoxylin (Sigma). After dehydration, slides were mounted using Pertex (HistoLab). Images were taken with a Leica TCS-SP2.

### ROC curves, survival analysis, and univariate and multivariate models

Predicted probabilities for CCS3 affiliation were calculated using a binary logistic regression with miR-200ba429 and miR-200c141 methylation as covariates. The x-beta values for predicting recurrence were calculated using a Cox regression with miR-200ba429 and miR-200c141 methylation as covariates. The sensitivity and specificity of CCS3 and recurrence prediction were calculated by plotting the receiver operating characteristic (ROC) curve of the predicted probabilities (binary logistic regression) and x-beta values (Cox regression), respectively, and calculating the area under the curve (AUC) using IBM SPSS Statistics version 21 software.

Survival curves were plotted using the Kaplan-Meier method and significance was evaluated by log-rank tests. DFS was measured from the day of surgery until detection of recurrence. Based on the above-described Cox regression model comprising 89 patients of the AMC-AJCCII-90 set (Fig. 6) the x-beta value 0.293 was chosen as cut-off for dividing the patients in a methylation low (< 0.293) and a methylation high (> 0.293) group, resulting in the prediction of recurrences with a sensitivity of 68.4% and a specificity of 75.7%. For the set comprising MSS patients only (fig. S12) the x-beta value -0.058 was chosen as cut-off (sensitivity 78.6% and specificity 56%). Univariate and multivariate Cox regression models were used to test the prognostic relevance of clinical factors and miR-200 methylation. The variables were selected based on being known risk factors of poor prognosis in stage II colon cancer and dichotomized as follows: gender (male vs female), age at operation (mean-dichotomized, <70.24 vs >70.24), location (right-vs left-sided), differentiation grade (well and moderate vs poor), T-stage (T3 vs T4), MSI vs MSS, BRAFV600E and MSS vs the rest of the patients, miR-200ba429 + miR-200c141 methylation (predicted probability of < 0.293 vs > 0.293).

For three patients of the AMC-AJCCII-90 dataset no information about differentiation grade was available, so these patients were left out of the uni- and multivariate analyses. The patient for whom miR-200 promoter methylation could not be assessed due to insufficient gDNA quality belonged to these three patients, thus, 87 patients were included in these analyses.

### Statistical Analysis

Hypergeometric tests were used to determine the significance of association of subtypes with molecular or clinical features in the CIT set (Fig. 1B) and of the overrepresentation of EMT genes in miRNA regulons (fig. S4D). The log-rank test was used to compare the DFS of different groups in KM curves. The correlation of miR-200 methylation and miR-200 expression was performed using linear regression and the P-values were based on Pearson correlation. For all other comparisons, two-tailed Student's t-tests were used. If not otherwise indicated mean + SD is depicted. If applicable, the Benjamini-Hochberg method was used to correct for multiple hypothesis testing.

### REFERENCES

1. A. Schlicker, G. Beran, C. M. Chresta, G. McWalter, A. Pritchard, S. Weston, S. Runswick, S. Davenport, K. Heathcote, D. A. Castro, G. Orphanides, T. French, L. F. Wessels, Subtypes of primary colorectal tumors correlate with response to targeted treatment in colorectal cell lines. BMC medical genomics 5, (2012).
2. F. De Sousa E Melo, X. Wang, M. Jansen, E. Fessler, A. Trinh, L. P. M. H. de Rooij, J. H. de Jong, O. J. de Boer, R. van Leersum, M. F. Bijlsma, H. Rodermond, M. van der Heijden, C. J. M. van Noesel, J. B. Tuynman, E. Dekker, F. Markowetz, J. P. Medema, L. Vermeulen, Poor-prognosis colon cancer is defined by a molecularly distinct subtype and develops from serrated precursor lesions. Nature medicine 19, 614-618 (2013).
3. A. Sadanandam, C. A. Lyssiotis, K. Homicsko, E. A. Collisson, W. J. Gibb, S. Wullschleger, L. C. G. Ostos, W. A. Lannon, C. Grotzinger, M. Del Rio, B. Lhermitte, A. B. Olshen, B. Wiedenmann, L. C. Cantley, J. W. Gray, D. Hanahan, A colorectal cancer classification system that associates cellular phenotype and responses to therapy. Nature medicine 19, 619-625 (2013).
4. E. Budinska, V. Popovici, S. Tejpar, G. D'Ario, N. Lapique, K. O. Sikora, A. F. Di Narzo, P. Yan, J. G. Hodgson, S. Weinrich, F. Bosman, A. Roth, M. Delorenzi, Gene expression patterns unveil a new level of molecular heterogeneity in colorectal cancer. The Journal of pathology 231, 63-76 (2013).
5. P. Roepman, A. Schlicker, J. Tabernero, I. Majewski, S. Tian, V. Moreno, M. H. Snel, C. M. Chresta, R. Rosenberg, U. Nitsche, T. Macarulla, G. Capella, R. Salazar, G. Orphanides, L. F. Wessels, R. Bernards, I. M. Simon, Colorectal cancer intrinsic subtypes predict chemotherapy benefit, deficient mismatch repair and epithelial-to-mesenchymal transition. International journal of cancer 134, 552-562 (2014).
6. L. Marisa, A. de Reyniès, A. Duval, J. Selves, M. P. Gaub, L. Vescovo, M.-C. Etienne-Grimaldi, R. Schiappa, D. Guenot, M. Ayadi, S. Kirzin, M. Chazal, J.-F. Fléjou, D. Benchimol, A. Berger, A. Lagarde, E. Pencreach, F. Piard, D. Elias, Y. Parc, S. Olschwang, G. Milano, P. Laurent-Puig, V. Boige, Gene expression classification of colon cancer into molecular subtypes: characterization, validation, and prognostic value. PLoS medicine 10, e1001453-e1001453 2013).
7. X. Wang, F. Markowetz, F. De Sousa E Melo, J. P. Medema, L. Vermeulen, Dissecting cancer heterogeneity--an unsupervised classification approach. Int J Biochem Cell Biol 45, 2574-2579 (2013).
8. M. S. Carro, W. K. Lim, M. J. Alvarez, R. J. Bollo, X. Zhao, E. Y. Snyder, E. P. Sulman, S. L. Anne, F. Doetsch, H. Colman, A. Lasorella, K. Aldape, A. Califano, A. Iavarone, The transcriptional network for mesenchymal transformation of brain tumours. Nature 463, 318-325 (2010).
9. D. Yang, Y. Sun, L. Hu, H. Zheng, P. Ji, C. V. Pecot, Y. Zhao, S. Reynolds, H. Cheng, R. Rupaimoole, D. Cogdell, M. Nykter, R. Broaddus, C. Rodriguez-Aguayo, G. Lopez-Berestein, J. Liu, I. Shmulevich, A. K. Sood, K. Chen, W. Zhang, Integrated analyses identify a master microRNA regulatory network for the mesenchymal subtype in serous ovarian cancer. Cancer cell 23, 186-199 (2013).
10. F. Song, D. Yang, B. Liu, Y. Guo, H. Zheng, L. Li, T. Wang, J. Yu, Y. Zhao, R. Niu, H. Liang, H. Winkler, W. Zhang, X. Hao, K. Chen, Integrated microRNA network analyses identify a poor-prognosis subtype of gastric cancer characterized by the miR-200 family. Clin Cancer Res 20, 878-889 (2014).
11. D. P. Bartel, MicroRNAs: Genomics, Biogenesis, Mechanism, and Function. Cell 116, 281-297 (2004).
12. M. Selbach, B. Schwanhäusser, N. Thierfelder, Z. Fang, R. Khanin, N. Rajewsky, Widespread changes in protein synthesis induced by microRNAs. Nature 455, 58-63 (2008).
13. D. Baek, J. Villén, C. Shin, F. D. Camargo, S. P. Gygi, D. P. Bartel, The impact of microRNAs on protein output. Nature 455, 64-71 (2008).
14. M. S. Nicoloso, R. Spizzo, M. Shimizu, S. Rossi, G. A. Calin, MicroRNAs - the micro steering wheel of tumour metastases. Nature Reviews Cancer 9, 293-302 (2009).
15. J. P. Thiery, H. Acloque, R. Y. J. Huang, M. A. Nieto, Epithelial-mesenchymal transitions in development and disease. Cell 139, 871-890 (2009).
16. P. A. Gregory, A. G. Bert, E. L. Paterson, S. C. Barry, A. Tsykin, G. Farshid, M. A. Vadas, Y. Khew-Goodall, G. J. Goodall, The miR-200 family and miR-205 regulate epithelial to mesenchymal transition by targeting ZEB1 and SIP1. Nature cell biology 10, 593-601 (2008).
17. S.-M. Park, A. B. Gaur, E. Lengyel, M. E. Peter, The miR-200 family determines the epithelial phenotype of cancer cells by targeting the E-cadherin repressors ZEB1 and ZEB2. Genes & development 22, 894-907 (2008).
18. R. Neves, C. Scheel, S. Weinhold, E. Honisch, K. M. Iwaniuk, H.-I. Trompeter, D. Niederacher, P. Wernet, S. Santourlidis, M. Uhrberg, Role of DNA methylation in miR-200c/141 cluster silencing in invasive breast cancer cells. BMC research notes 3, (2010).
19. L. Vrba, T. J. Jensen, J. C. Garbe, R. L. Heimark, A. E. Cress, S. Dickinson, M. R. Stampfer, B. W. Futscher, Role for DNA methylation in the regulation of miR-200c and miR-141 expression in normal and cancer cells. PloS one 5, e8697-e8697 (2010).
20. E. D. Wiklund, J. B. Bramsen, T. Hulf, L. Dyrskjøt, R. Ramanathan, T. B. Hansen, S. B. Villadsen, S. Gao, M. S. Ostenfeld, M. Borre, M. E. Peter, T. F. Ørntoft, J. Kjems, S. J. Clark, Coordinated epigenetic repression of the miR-200 family and miR-205 in invasive bladder cancer. International Journal of Cancer 128, 1327-1334 (2011).
21. V. Davalos, C. Moutinho, a. Villanueva, R. Boque, P. Silva, F. Carneiro, M. Esteller, Dynamic epigenetic regulation of the microRNA-200 family mediates epithelial and mesenchymal transitions in human tumorigenesis. Oncogene 31, 2062-2074 (2012).
22. T. Sugai, W. Habano, Y.-F. Jiao, M. Tsukahara, Y. Takeda, K. Otsuka, S.-I. Nakamura, Analysis of Molecular Alterations in Left- and Right-Sided Colorectal Carcinomas Reveals Distinct Pathways of Carcinogenesis. The Journal of Molecular Diagnostics 8, 193-201 (2006).
23. S. D. Markowitz, M. M. Bertagnolli, Molecular Basis of Colorectal Cancer. New England Journal of Medicine 361, 2449-2460 (2009).
24. N. The Cancer Genome Atlas, Comprehensive molecular characterization of human colon and rectal cancer. Nature 487, 330-337 (2012).
25. S. Brabletz, T. Brabletz, The ZEB/miR-200 feedback loop--a motor of cellular plasticity in development and cancer? EMBO reports 11, 670-677 (2010).
26. E. N. Howe, D. R. Cochrane, J. K. Richer, Targets of miR-200c mediate suppression of cell motility and anoikis resistance. Breast cancer research : BCR 13, R45-R45 (2011).
27. U. Burk, J. Schubert, U. Wellner, O. Schmalhofer, E. Vincan, S. Spaderna, T. Brabletz, A reciprocal repression between ZEB1 and members of the miR-200 family promotes EMT and invasion in cancer cells. EMBO reports 9, 582-589 (2008).
28. M. Shioiri, T. Shida, K. Koda, K. Oda, K. Seike, M. Nishimura, S. Takano, M. Miyazaki, Slug expression is an independent prognostic parameter for poor survival in colorectal carcinoma patients. British journal of cancer 94, 1816-1822 (2006).
29. S. Spaderna, O. Schmalhofer, F. Hlubek, G. Berx, A. Eger, S. Merkel, A. Jung, T. Kirchner, T. Brabletz, A transient, EMT-linked loss of basement membranes indicates metastasis and poor survival in colorectal cancer. Gastroenterology 131, 830-840 (2006).
30. J. H. Taube, J. I. Herschkowitz, K. Komurov, A. Y. Zhou, S. Gupta, J. Yang, K. Hartwell, T. T. Onder, P. B. Gupta, K. W. Evans, B. G. Hollier, P. T. Ram, E. S. Lander, J. M. Rosen, R. A. Weinberg, S. A. Mani, Core epithelial-to-mesenchymal transition interactome gene-expression signature is associated with claudin-low and metaplastic breast cancer subtypes. Proceedings of the National Academy of Sciences 107, 15449-15454 (2010).
31. A. Subramanian, P. Tamayo, V. K. Mootha, S. Mukherjee, B. L. Ebert, M. A. Gillette, A. Paulovich, S. L. Pomeroy, T. R. Golub, E. S. Lander, J. P. Mesirov, Gene set enrichment analysis : A knowledge-based approach for interpreting genome-wide expression profiles. Proceedings of the National Academy of Sciences 102, 15545-15550 (2005).
32. C. J. Gröger, M. Grubinger, T. Waldhör, K. Vierlinger, W. Mikulits, Meta-analysis of gene expression signatures defining the epithelial to mesenchymal transition during cancer progression. PloS one 7, e51136-e51136 (2012).
33. A. Sadanandam, X. Wang, F. de Sousa E Melo, J. W. Gray, L. Vermeulen, D. Hanahan, J. P. Medema, Reconciliation of classification systems defining molecular subtypes of colorectal cancer: interrelationships and clinical implications. Cell Cycle 13, 353-357 (2014).
34. J. P. Thiery, Epithelial-mesenchymal transitions in tumour progression. Nature Reviews Cancer 2, 442-454 (2002).
35. A. D. Rhim, E. T. Mirek, N. M. Aiello, A. Maitra, J. M. Bailey, F. McAllister, M. Reichert, G. L. Beatty, A. K. Rustgi, R. H. Vonderheide, S. D. Leach, B. Z. Stanger, EMT and dissemination precede pancreatic tumor formation. Cell 148, 349-361 (2012).
36. A. D. Rhim, F. I. Thege, S. M. Santana, T. B. Lannin, T. N. Saha, S. Tsai, L. R. Maggs, M. L. Kochman, G. G. Ginsberg, J. G. Lieb, V. Chandrasekhara, J. A. Drebin, N. Ahmad, Y.-X. Yang, B. J. Kirby, B. Z. Stanger, Detection of circulating pancreas epithelial cells in patients with pancreatic cystic lesions. Gastroenterology 146, 647-651 (2014).
37. D. J. Weisenberger, K. D. Siegmund, M. Campan, J. Young, T. I. Long, M. A. Faasse, G. H. Kang, M. Widschwendter, D. Weener, D. Buchanan, H. Koh, L. Simms, M. Barker, B. Leggett, J. Levine, M. Kim, A. J. French, S. N. Thibodeau, J. Jass, R. Haile, P. W. Laird, CpG island methylator phenotype underlies sporadic microsatellite instability and is tightly associated with BRAF mutation in colorectal cancer. Nature genetics 38, 787-793 (2006).
38. S. Ogino, M. Cantor, T. Kawasaki, M. Brahmandam, G. J. Kirkner, D. J. Weisenberger, M. Campan, P. W. Laird, M. Loda, C. S. Fuchs, CpG island methylator phenotype (CIMP) of colorectal cancer is best characterised by quantitative DNA methylation analysis and prospective cohort studies. Gut 55, 1000-1006 (2006).
39. M. N. McCall, B. M. Bolstad, R. A. Irizarry, Frozen robust multiarray analysis (fRMA). Biostatistics 11, 242-253 (2010).
40. W. E. Johnson, C. Li, A. Rabinovic, Adjusting batch effects in microarray expression data using empirical Bayes methods. Biostatistics 8, 118-127 (2007).
41. H. M. Zhang, H. Chen, W. Liu, H. Liu, J. Gong, H. Wang, A. Y. Guo, AnimalTFDB: a comprehensive animal transcription factor database. Nucleic Acids Res 40, D144-149 (2012).
42. A. A. Margolin, I. Nemenman, K. Basso, C. Wiggins, G. Stolovitzky, R. Dalla Favera, A. Califano, ARACNE: an algorithm for the reconstruction of gene regulatory networks in a mammalian cellular context. BMC Bioinformatics 7 Suppl 1, S7 (2006).
43. R. A. Irizarry, B. M. Bolstad, F. Collin, L. M. Cope, B. Hobbs, T. P. Speed, Summaries of Affymetrix GeneChip probe level data. Nucleic Acids Res 31, e15 (2003).
44. F. Hong, R. Breitling, C. W. McEntee, B. S. Wittner, J. L. Nemhauser, J. Chory, RankProd: a bioconductor package for detecting differentially expressed genes in meta-analysis. Bioinformatics 22, 2825-2827 (2006).

## Claims

**1.** Method of diagnosing, prognosticating, predicting or detecting pre-cancer, colorectal cancer (CRC) tumor progression, or metastasis in a human subject comprising the steps of:
(a) determining in a biological sample from said human subject the methylation status of at least one CpG in the miR-200ba429 promoter region and/or the miR-200c141 promoter region, and
(b) determining the methylation status of one or more of said at least one CpG,
(c) determining the risk of developing CRC, metastasis and/or tumor relapse based on said methylation status, wherein hypermethylation of said at least one CpG indicates an increased risk of developing CRC, metastasis and/or tumor relapse.

**2.** Method of determining whether a colorectal cancer cell has an epithelial or mesenchymal phenotype comprising the steps of:
(a) determining in a biological sample from said human subject the methylation status of at least one CpG in the in the miR-200ba429 promoter region and/or the miR-200c141 promoter region, and
(b) determining the methylation status of one or more of said at least one CpG,
(c) determining the cancer cells as having epithelial or mesenchymal phenotype based on said methylation status, wherein hypermethylation of said at least one CpG indicates a mesenchymal phenotype.

**2.** The method according to claim 1 or 2, wherein said methylation status comprises the presence or absence of methylation of DNA at said at least one CpG.

**3.** The method according to any one of claims 1 or 3, wherein said biological sample comprises cancer cells from a primary tumor.

**4.** Method according to any one of claims 1-3, wherein hypermethylation is determined in a CpG of the miR-200ba429 promoter region and in a CpG of the miR-200c141 promoter region.

**5.** Method according to any one of claims 1, 3 and 4, wherein said human subject is suffering from colorectal cancer in stage I or II.

**6.** Method of predicting the response in a human subject to treatment with a demethylating agent comprising:
(a) determining the risk of developing metastasis and/or tumor relapse in human subject according to the method of any one of claims 1, 3-5 or the phenotype of a cancer cells in a sample from said colorectal cancer patient according to any one of claims 2-5, and
(b) predicting the clinical response to said treatment, based on the outcome of step (a).

**7.** A demethylating agent for use in the treatment of human subject with hypermethylation of the miR-200ba429 promoter region and/or the miR-200c141 promoter region.

**8.** Method according to claim 6 or the demethylating agent for use according to claim 7, wherein said demethylating agent is 5-aza-cytidine, 5-aza-2'-deoxycytidine (decitabine), zebularine [1-(β-D-ribofuranosyl(dihydro-pyrimidin-2-1)] or L-methionine.

**9.** A kit for assessing methylation in a test sample, comprising in a package:
A reagent that (a) modifies methylated cytosine residues but not non-methylated cytosine residues, or that (b) modifies non-methylated cytosine residues but not methylated cytosine residues; and one or more oligonucleotide primers and/or pair of oligonucleotide primers that specifically hybridizes under amplification conditions to a nucleic acid selected from the group consisting of the miR-200ba429 promoter region and/or the miR-200c141 promoter region.
